(19) Europäisches Patentamt / European Patent Office / Office européen des brevets

(11) **EP 3 891 273 B1**

(12) **EUROPEAN PATENT SPECIFICATION**

(45) Date of publication and mention
of the grant of the patent:
**29.07.2026 Bulletin 2026/31**

(21) Application number: **19813009.8**

(22) Date of filing: **04.12.2019**

(51) International Patent Classification (IPC):
*C12N 7/00* (2006.01)    *C12N 15/867* (2006.01)
*A61K 48/00* (2006.01)

(52) Cooperative Patent Classification (CPC):
**C12N 7/00; A61K 48/0041; C12N 15/86;**
C12N 2740/10043; C12N 2740/16043

(86) International application number:
**PCT/EP2019/083637**

(87) International publication number:
**WO 2020/115114 (11.06.2020 Gazette 2020/24)**

(54) **VIRAL TRANSDUCTION USING POLOXAMINES**

VIRALE TRANSDUKTION UNTER VERWENDUNG VON POLOXAMINEN

TRANSDUCTION VIRALE À L'AIDE DE POLOXAMINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2018 EP 18210207**

(43) Date of publication of application:
**13.10.2021 Bulletin 2021/41**

(73) Proprietor: **Revvity Gene Delivery GmbH
82166 Gräfelfing (DE)**

(72) Inventors:
• **THIRION, Christian
82152 Martinsried (DE)**
• **HASENÖDER, Stefan
80997 München (DE)**
• **SCHRÖDEL, Silke
82152 Martinsried (DE)**

(74) Representative: **Dentons Patent Solutions
Rechtsanwaltsgesellschaft mbH
Jungfernturmstraße 2
80333 München (DE)**

(56) References cited:
**WO-A1-2013/127964    WO-A1-2017/019905**

• **ANA REY-RICO ET AL: "PEO-PPO-PEO micelles as effective rAAV-mediated gene delivery systems to target human mesenchymal stem cells without altering their differentiation potency", ACTA BIOMATERIALIA, vol. 27, 28 August 2015 (2015-08-28), AMSTERDAM, NL, pages 42 - 52, XP055561289, ISSN: 1742-7061, DOI: 10.1016/j.actbio.2015.08.046**
• **EMA C. CIUCUREL ET AL: "A Poloxamine-Polylysine Acrylate Scaffold for Modular Tissue Engineering", JOURNAL OF BIOMATERIALS SCIENCE. POLYMER EDITION., vol. 22, no. 18, 1 January 2011 (2011-01-01), NL, pages 2515 - 2528, XP055654592, ISSN: 0920-5063, DOI: 10.1163/092050610X541133**
• **HOWARD E. DAVIS ET AL: "Charged Polymers Modulate Retrovirus Transduction via Membrane Charge Neutralization and Virus Aggregation", BIOPHYSICAL JOURNAL, vol. 86, no. 2, 1 February 2004 (2004-02-01), AMSTERDAM, NL, pages 1234 - 1242, XP055562198, ISSN: 0006-3495, DOI: 10.1016/S0006-3495(04)74197-1**
• **LEE H J ET AL: "Retronectin enhances lentivirus-mediated gene delivery into hematopoietic progenitor cells", BIOLOGICALS, ACADEMIC PRESS LTD., LONDON, GB, vol. 37, no. 4, 1 August 2009 (2009-08-01), pages 203 - 209, XP026348636, ISSN: 1045-1056, [retrieved on 20090304]**

**(Cont. next page)**

• GARRETT C. HEFFNER ET AL: "Prostaglandin E2 Increases Lentiviral Vector Transduction Efficiency of Adult Human Hematopoietic Stem and Progenitor Cells", MOLECULAR THERAPY : THE JOURNAL OF THE AMERICAN SOCIETY OF GENE THERAPY, vol. 26, no. 1, 1 January 2018 (2018-01-01), US, pages 320 - 328, XP055561352, ISSN: 1525-0016, DOI: 10.1016/j.ymthe.2017.09.025

• PETRILLO CAROLINA ET AL: "Cyclosporine H Overcomes Innate Immune Restrictions to Improve Lentiviral Transduction and Gene Editing In Human Hematopoietic Stem Cells", CELL STEM CELL, vol. 23, no. 6, 8 November 2018 (2018-11-08), pages 820, XP085555974, ISSN: 1934-5909, DOI: 10.1016/J.STEM.2018.10.008

• GRETCHEN LEWIS ET AL: "Staurosporine Increases Lentiviral Vector Transduction Efficiency of Human Hematopoietic Stem and Progenitor Cells", MOLECULAR THERAPY - METHODS & CLINICAL DEVELOP, vol. 9, 5 April 2018 (2018-04-05), GB, pages 313 - 322, XP055592948, ISSN: 2329-0501, DOI: 10.1016/j.omtm.2018.04.001

**Description**

The present invention relates to a composition comprising or consisting of (a) a poloxamine; and (b)(i) retroviral vector; and/or (ii) a vertebrate cell.

In this specification, a number of documents including patent applications and manufacturer's manuals are cited.

For genetic modifications of most cells, in particular primary cells, transduction mediated by viral vectors is a preferred method. In particular retroviral vectors such as for example lentiviral vectors are used. Lentiviral (LV) vectors pseudotyped with glycoproteins of vesicular stomatitis virus (VSV-G) stably integrate into the chromosomes of both proliferating and non-proliferating cells (Bukrinsky et al. 1993; Burns et al. 1993; Funke et al. 2008). Improved safety features, such as a split-genome lentiviral packaging design of third generation and deletions in the 3' long terminal repeat (LTR) of the transfer vector, has made LV a system favored by many researchers (Dull et al. 1998). Lentivirus-transduced cells barely show activation of stress signal pathways or any other phenotypic alteration and therefore promise a multitude of gene therapy and immunotherapy applications (Gruber et al. 2000; Rouas et al. 2002).

Currently, there is great variation regarding transduction conditions for efficient LV gene delivery into target cells used among research and clinical groups (Millington et al. 2009). Normal lymphocytes and primary tumors, as well as cell lines of the lymphoid lineage are known to be difficult to transduce efficiently (Anastasov et al. 2009) (Anastasov et al. 2010). High gene transfer rates into primary cells and the ability to produce high titers of virus particles for large-scale transduction of patient cells are prerequisites for clinical trials. As large-scale production of these viruses is extremely complex and expensive (Wurm et al. 2010), every improvement of lentiviral transduction rates will lead to a reduction in the need for virus production and will further help to reduce the costs of clinical trials.

Higher efficiency of gene transfer can be achieved by a variety of different strategies: The concentration of virus supernatants by ultracentrifugation (Burns et al. 1993) or by ultrafiltration (Anastasov et al. 2009) is one possible way to improve the efficiency of gene transfer. Another frequently used strategy to enhance gene transfer rates is the supplementation of different agents such are polycations or cationic liposomes. However, most of these adjuvant treatments are toxic for the cells, limiting their use with sensitive target cells of primary origin (Wurm et al. 2010). Among other candidates, polybrene (a linear polycationic polymer) improved gene transduction rates in a broad range of target cells and became a leading transduction adjuvant in the field (Castro et al. 1988; Hesse, Ebbesen, and Kristensen 1978). Unfortunately, polybrene can be used only in short application times and at concentrations below 10 $\mu$g/ml (dependent on the target cell type) to avoid cellular toxicity and/or significant modulation of the transmembrane potential (Aubin, Weinfeld, and Paterson 1988) which limits its use in sensitive cells, such as hematopoietic cells.

Another common method to enhance viral transduction efficiency is the use of Retronectin, a recombinant human fibronectin fragment which promotes colocalization of lentivirus and target cell (Lee et al. 2009).

Vectofusin-1 is a relatively new viral transduction enhancer. It is a histidine-rich cationic amphipathic peptide derived from the LHA4 peptide family (Fenard et al. 2013). It acts by promoting the adhesion and fusion between viral and cellular membranes and was shown to improve transduction of human hematopoietic stem and progenitor cells (HSPCs) (Fenard et al. 2013; Ingrao et al. 2014).

Furthermore, centrifugation inoculation (frequently termed "spinoculation") of transduced cells directly after adding virus to the cells can also enhance transduction (Guo et al. 2011). It was demonstrated that centrifugation triggers dynamic actin and cofilin activity, which leads to upregulation of the HIV-1 receptor and co-receptor CD4 and CXCR4 (Guo et al. 2011). Spinoculation can also be combined with the use of transduction enhancers to further increase transduction efficiency.

Yet another method to deliver therapeutically relevant substances to cells is to package them into biomacromolecules or polymers. For proteins like Wnt it has been demonstrated that packaging into liposomes or phospholipids and cholesterol enhances stability and activity and thus improves biological activity on the target cells (Morrell et al. 2008; Tüysüz et al. 2017). Non viral transfer of nucleic acids into cells was also successfully achieved by using linear (Kabanov, Zhu, and Alakhov 2005; Lemieux and Guerin 2000; Mahajan et al. 2017; Song et al. 2014) and branched amphiphilic co-polymers (Zhang et al. 2009). Furthermore, various polymers have been proven helpful to solubilize hydrophobic drugs in micelles to enable delivery into target cells (Alakhov et al. 1999; Alvarez-Lorenzo et al. 2010; Chiappetta and Sosnik 2007; Erukova et al. 2000). These Polymeric substances include, amongst others, polyethylene glycol (PEG), poly(propylene oxide) (PPO), poly(D,L-lactic acid) (PDLLA), Poloxamers and Poloxamines. The encapsulation of drugs within the hydrophobic core of the micelle significantly increases solubility, stability and uptake into the target cell.

Complexes of Cas9 protein sgRNA ribonucleoprotein (RNP) have proven valuable for efficient gene modification while minimizing potential off-target effects (Gundry et al. 2016) Non-viral polymeric delivery systems like poloxamines and other polymers could serve as valuable tools to deliver RNPs into target cells to enable correction of genetic defects in vitro and in vivo (Khan et al. 2016).

Further non-retroviral vectors have been developed recently and are used for gene transfer Vectors based on enveloped herpes viruses including HSV-1, rhesus CMV (RhCMV), MCMV received high attention for their neuronal gene transfer capacity, and good properties as vaccine carriers (Hansen et al. 2011; Marconi, Manservigi, and Epstein 2010;

Mohr et al. 2010). Recently, viral vectors derived from the Lymphocytic choriomeningitis (LCM) virus belonging to the family of Arenviridae induced a potent immune response in homologous and heterologous vaccination regimens (Wingerath et al. 2017).

WO 2017/019905 A1 describes methods, compositions, vectors, and kits comprising an antagonist of a truncated TrkC or a truncated TrkB. Also described are methods of treating and/or preventing an otic disease or condition associated with an elevated expression level of a truncated TrkC or truncated TrkB isoform.

Ana Rey-Rico et al. describes in Acta Biomaterialia, vol. 27, 28 August 2015, on pages 42-52 PEO-PPO-PEO micelles as effective rAAV-mediated gene delivery systems to target human mesenchymal stem cells without altering their differentiation potency.

MA C. Ciucurel et al. describes in Journal of Biomaterials Science, Polymer edition, vol. 22, no. 18, 1 January 2011, on pages 2515-2528 a Poloxamine-Polylysine acrylate scaffold for modular tissue engineering.

Howard E. Davis et al. describes in Biophysical Journal, vol. 86, no. 2, 1 February 2004, on pages 1234-1242 charged polymers that modulate retrovirus transduction via membrane charge neutralization and virus aggregation.

Lee H J et al. describes in Biologicals, Academic Press LTD vol. 37, no. 4, 1 August 2009, on pages 203-209 that retronectin enhances lentivirus-mediated gene delivery into hematopoietic progenitor cells.

Garrett C. Heffner et al. describes in Molecular Therapy: The Journal of the American Society of Gene Therapy, vol. 26, no. 1, 1 January 2018, on pages 320-328 that prostaglandin E2 increases lentiviral vector transduction efficiency of adult human hematopoietic stem and progenitor cells.

WO 2013/127964 A1 describes a method for transducing a target cell, the method comprising the step of contacting a target cell with a retroviral vector and a poloxamer having a molecular weight of 12,8 kDa to about 15 kDa.

Petrillo Carolina et al. describes in Cell Stem Cell, vol. 23, no. 6, 8 November 2018, on page 820 that cyclosporine H overcomes innate immune restrictions to improve lentiviral transduction and gene editing in human hematopoietic stem cells.

Gretchen Lewis et al. describes in Molecular Therapy, vol. 9, 5 April 2018, on pages 313-322 than staurosporine increases lentiviral vector transduction efficiency of human hematopoietic stem and progenitor cells.

The technical problem underlying the present invention was to identify improved means and methods for transducing cells with viral vectors.

The solution to this technical problem is achieved by providing the embodiments characterized in the claims.

The present invention is defined by independent claims. The dependent claims depict other embodiments of the invention.

Accordingly, the present invention, in a first aspect, relates to a composition comprising or consisting of (a) a poloxamine; and (b)(i) a retroviral vector; and/or (ii) a vertebrate cell.

Related to the first aspect, the present invention provides a composition comprising or consisting of (a) a poloxamine; and (b)(i) an enveloped viral vector; and/or (ii) a vertebrate cell.

Also related to the first aspect, the present invention provides a composition comprising or consisting of (a) a poloxamine; (b) (i) a retroviral vector; and (ii) a vertebrate cell.

Furthermore, the present invention provides a composition comprising or consisting of (a) a poloxamine; (b) (i) a retroviral vector; and (ii) a vertebrate cell, wherein said poloxamine is provided in a concentration in the range from about 0.1 to about 40 mg/ml.

The term "poloxamine" has its art-established meaning. It refers to an amphiphilic block copolymer comprising a central 1,2-ethylenediamine, wherein two valances of either nitrogen atom are substituted with moieties each comprising a plurality of ethylene oxide and/or propylene oxide building blocks. To explain further, said moieties contain a multitude of ethylene oxide and/or propylene oxide building blocks connected by ether bonds. Preferred embodiments thereof are shown in formula (I) further below. It is preferred that each of these moieties contains both ethylene oxide and propylene oxide building blocks. There is no requirement in accordance with the present invention in that respect, though. To the extent the building block comprises both ethylene oxide and propylene oxide, preference is given to moieties which are divided in two parts, namely a part which consists exclusively of propylene oxide building blocks and a second part which consists exclusively of ethylene oxide building blocks. Again, there is no requirement in accordance with the invention in that respect. Accordingly, it is also conceivable that one or more moieties are chains of alternating ethylene oxide and propylene oxide building blocks, or that there is a random sequence of ethylene oxide and propylene oxide building blocks.

To the extent said moieties have the above defined bipartite structure, i.e. a part which consists exclusively of ethylene oxide monomers and a second part which consists exclusively of propylene oxide building blocks, preference is given to the part consisting of propylene oxide building blocks being bound directly to the nitrogen atoms, and the part consisting of ethylene oxide building blocks being located at a position distal from the nitrogen atoms (also known as "sequential poloxamines"). Also this feature is illustrated in the preferred embodiment of formula (I) shown further below.

While not being particularly preferred, it is still within the ambit of the present invention that the four moieties as defined above within a single poloxamine molecule each have a distinct structure. To give an example, all four moieties could be (different) random copolymers of ethylene oxide and propylene oxide building blocks. In another example, two moieties could have a bipartite structure with a nitrogen proximal part consisting of propylene oxide building blocks and a nitrogen distal part consisting of ethylene oxide building blocks, and two further moieties could consist exclusively of either ethylene oxide building blocks or exclusively of propylene oxide building blocks.

In the class of molecules described above, three valances on the nitrogen are occupied. It is within the ambit of the present invention that four valances on the nitrogen are occupied and the nitrogen carries a positive charge. In that case, one or more counter ions are preferably present, thereby rendering the composition of matter electroneutral. Moieties occupying the fourth valance on one or both nitrogens of said poloxamine are preferably hydrogen, lower alkyl, lower alkenyl or lower alkynyl. The term "lower" in this context preferably refers to $C_1$ to $C_6$, ($C_2$ to $C_6$ in case of alkenyl and alkynyl) preferably $C_1$ to $C_4$ ($C_2$ to $O_4$ in case of alkenyl and alkynyl). Particularly preferred alkyl moieties are methyl, ethyl, n-propyl and i-propyl. The moieties occupying the fourth valance on both nitrogen atoms, in case both nitrogen atoms are quaternary are preferably identical. Particularly preferred is that both nitrogens are quaternary, and the corresponding moieties are methyl in either case.

Further envisaged moieties occupying the fourth valance on one or both nitrogens include cycloalkyl including cyclohexyl, aryl, arylalkyl, heteroaryl and heteroarylalkyl. A preferred aryl group is phenyl. Preferred alkyl groups in aralkyl and heteroarylalkyl are methyl and ethyl. Preferred heteroaryl groups are six-membered rings with one, two or three heteroatoms independently selected from N, S and O. Anyone of cycloalkyl, aryl, aralkyl, heteroaryl and heteroarylakyl may be substituted or unsubstituted. Preferred substituents include lower alkyl, lower alkoxy and halogen. "Lower" in this context has the same meaning as defined above ($C_1$ to $O_6$, preferably $C_1$ to $O_4$). Halogens include fluorine, chlorine and bromine.

The composition may comprise one poloxamine. It may also comprise two, three, four, five or more different poloxamines.

Preferred counter ions are halogenoids including fluoride, chloride, bromide and iodide. Further preferred counter ions include sulfate, phosphate, acetate as well as anions of other monocarboxylic acids, such as formide, propionate and butyrate. Salicylate is another envisaged counter ion. Preferred are pharmaceutically acceptable counter ions.

In the broadest definition, the ratio between ethylene oxide and propylene oxide building blocks is not particularly limited. In preferred embodiments, said ratio is between about 0,05 and about 1,5. More preferred propylene oxide-ethylene oxide ratios are disclosed further below in conjunction with a preferred embodiment. They apply mutatis mutandis to the broadest definition of poloxamines in accordance with the present invention.

Poloxamines have been considered as agents for formulating small molecule drugs or plain DNA plasmids. The prior art is silent, though, as regards the use of poloxamines for transducing enveloped viruses including retroviruses and lentiviruses. The results presented herein show a surprising specific high activity of poloxamines for enhancing transduction of vertebrate cells with lenti- and retrovirus vectors.

As is apparent from the remainder of the disclosure, said poloxamine acts as a transduction enhancer in accordance with the invention. The poloxamine does not serve as an excipient, dispersing agent or viscosity modulating agent.

Optionally, said composition is free of other transduction enhancers, i.e. it comprises only one or more poloxamines as regards agents which enhance transduction. In the alternative, further transduction enhancers, in particular transduction enhancers which belong to compound classes other than poloxamines may be present. This is the subject of preferred embodiments disclosed further below.

Said composition, to the extent poloxamines are considered, is a solution. In other words, it is not a gel.

Also, it is understood that said composition is isolated. In other words, it is not a composition comprising cells which would form only, e.g., inside the human or animal body upon administration of a corresponding composition which does not contain cells.

A viral vector is a particle which is derived from a naturally occurring virus. A naturally occurring virus generally comprises nucleic acid and proteins. An enveloped virus furthermore includes a lipid bilayer which typically originates from the host cell and generally comprises a viral envelope protein. A minimal requirement for a viral vector in accordance with the invention is said lipid bilayer with said viral envelope protein. Nucleic acids and/or further proteins may, but do not have to be present. The term "nucleic acid" refers to original viral nucleic acid, heterologous nucleic acid or a combination of both. In case of retroviral vectors, the viral nucleic acid is RNA. The nucleic acid may further comprise at least one long terminal repeat (LTR) sequence derived from a retrovirus and a packaging signal sequence. Optionally, the nucleic acid may comprise further genetic elements such as an RRE sequence. Further proteins, to the extent present, may include a reverse transcriptase, an integrase and capsid proteins. The integrase may be integration-deficient.

Accordingly, the terms "derivative" and "derived" refer to at least the following modification: introduction of a heterologous nucleic acid into the genetic material of the virus, or replacing all or most of the viral coding genes by heterologous nucleic acid sequences, and allowing for packing of said heterologous nucleic acid in viral particles. While being less preferred, a naturally occurring virus or virus-like particles devoid of any packaged virus-derived nucleic acids, is subsumed under the term "viral vector". An example thereof is a virus-like particle (VLP). Virus-like particles are non-infectious and contain neither viral genetic material nor heterologous nucleic acids. They are produced by expression of viral structural and/or accessory proteins, such as envelope or capsid proteins, which can result in the self-assembly of virus like particles (VLPs). Retrovirus and lentivirus-derived VLPs are used to deliver recombinant proteins and can be produced with high yields (Robert et al. 2017).

Exosomes are lipid bilayer-enclosed extracellular vehicles which can be used to transport proteins, nucleic acids and lipids between cells (Kowal, Tkach, and Thery 2014). These exosomes can also be pseudotyped with the viral glycoprotein VSV-G to improve delivery into mammalian cells (Meyer et al. 2017). Based on these properties it has been suggested that exosomes can be used in gene therapeutic applications (O'Loughlin, Woffindale, and Wood 2012).

Alternatively or in addition, protein components of the naturally occurring virus may be modified or exchanged. Examples thereof include pseudotyped retroviral vectors, for example, retrovirus vectors pseudotyped with VSV-G, a glycoprotein derived from the vesicular stomatitis virus (VSV) belonging to the family of Rhabdoviridae (for further details see below).

The term "enveloped" in relation to a virus refers to the presence of a lipid bilayer surrounding the virus particle. Generally, but not necessarily, one or more types of membrane proteins (transmembrane proteins, membrane-associated proteins and the like) are comprised in the envelope.

In a taxonomic sense and in accordance with the invention, the term "enveloped viruses" embraces the following DNA viruses: *Herpesviridae* and *Hepadnaviridae*. Furthermore, the term "enveloped viruses" embraces the following RNA viruses: *Retroviridae, Togaviridae, Arenaviridae, Flaviviridae, Orthomyxoviridae, Paramyxoviridae, Bunyaviridae, Rhabdoviridae, Filoviridae, Coronaviridae, Bornaviridae, Arteriviridae, Flaviviridae* and *Reoviridae.*

Viral vectors as such, including retroviral vectors, are known in the art. For example, retroviral vectors have been described in Retroviruses, Coffin JM, Hughes SH, Varmus HE, Cold Spring Harbor (NY): Cold Spring Harbour Laboratory Press; 1997; ISBN-10:0-87969-571-4. Briefly, retroviral vectors have the ability to integrate the genetic material they comprise into the host genome in a stable fashion. As is common to retroviruses, they contain a reverse transcriptase and, optionally, an integrase that enables RNA reverse transcription of RNA into complementary DNA (cDNA) and, optionally, integration into the genome of a target cell. Following cellular entry, the reverse transcriptase synthesizes viral cDNA and cDNA of the target sequence which is circularized and then inserted into the target cell's genome. While retroviral vectors can be replication competent, for safety reasons most retroviral vectors are designed to be replication defective. Corresponding viruses can still infect and deliver the genetic material to the target cell, but cannot enter the replicative cycle without e.g., helper proteins to provide the missing viral proteins for the production of new virions. This can be achieved by deleting or substituting genes necessary for virion replication and packaging. One way of rendering retroviral vectors replication defective is to remove the gag, pol and env genes which may be replaced by an expression cassette containing a target sequence for introduction into the genome of a target cell. The Long Terminal Repeats (LTR) and psi (T) elements are generally retained in order to allow transgene expression and packaging into viral capsids during vector production.

Conceivably, target sequences comprised in a vector as defined herein can be any sequences, in particular those allowing integration into the genome of the target cell. For example, protein or peptide coding sequences, regulatory sequences for RNA interference, miRNA expression or oncolytic viruses can be expressed in a cell after transduction, wherein the protein can be, e.g., a marker or a therapeutically valuable protein. The concept of expressing a therapeutic protein after stable integration via retroviral transduction is, embraced by the term "gene therapy" that is well known in the art. Also, target sequences may result upon transduction in the downregulation of proteins or non-coding RNA transcripts (by RNA interference) that have an effect in the transduced cells or organism receiving transduced cells. MicroRNAs (miRNAs) and small interfering RNAs (siRNAs) are functional small molecules that regulate the stability or translational efficiency of target messenger RNAs. Their actions include repression of protein synthesis and induction of targeted mRNA degradation (see, e.g. (Bartel 2009)).

In another preferred embodiment of the method of the invention, the retroviral vector is a lentiviral vector.

A lentiviral vector is a vector based on a lentivirus virion, i.e. a subclass of retroviruses that can integrate into the genome of non-dividing target cells being a unique feature of lentiviruses to have self inactivated (SIN) region of replication in contrast to other retroviral vectors. Lentiviruses are, e.g., described in detail in Retroviruses, Coffin JM, Hughes SH, Varmus HE, Cold Spring Harbor (NY): Cold Spring Harbour Laboratory Press; 1997; ISBN-10:0-87969-571-4; (Matrai, Chuah, and VandenDriessche 2010; O'Connell et al. 2010). A lentiviral vector can be based, e.g., on a lentivirus of the group of bovine, equine, feline, ovine/caprine or primate lentivirus group. Preferably, the lentiviral vector is based on a primate lentivirus such as, HIV1, HIV2 or SIV virus. Most preferred, the lentiviral vector is based on an HIV1 lentivirus. As the skilled person is aware, most (commercially available) lentiviral vectors represent a mixture of viral constituents from different viruses and are, hence, to some extent "hybrid" vectors. For example, a lentiviral vector may comprise constituents from HIV1 (Human immunodeficiency 1 virus), VSV (Vesicular Stomatitis Virus), CMV (Cytomegalovirus), WHV (Woodchuck Hepatitis *Virus* (WHP) viruses. Therefore, also pseudotyped vectors are envisaged in accordance with the invention.

In a more preferred embodiment, the lentiviral vector is pseudotyped with VSV-G; with an antibody fragment or protein scaffolds fused to VSV-G, such as e.g., CD3-VSV-G, CD4-VSV-G, CD8-VSV-G, CD34-VSV-G, CD30-VSV-G, CD44-VSV-G, EGFR-VSV-G; and/or the transferrin receptor (TnfR).

The term "pseudotyped" in the context of viral vectors is well known in the art and described for example in (Bischof and Cornetta 2010). Pseudotyping refers to the modulation of the cell type specificity of a viral vector by integration of foreign viral envelope glycoproteins (gp), including but not limited to RD114, GALV, LCMV, MLV, gp160, equine infectious anemia virus (EIAV) gp, feline immunodeficiency virus (FIV) gp, rabies virus glycoprotein (RV-G), fusion glycoprotein type E (FuG-E) gp (reviewed Curr Gene Ther. 5, 387-398 (2005)). Using this approach, host tropism can be altered and/or stability of the virus can be decreased or increased. For example, glycoprotein G of the Vesicular stomatitis virus (VSV-G) can be used for pseudotyping a lentiviral virus as described, e.g., in (Burns et al. 1993). The use of VSV-G advantageously enables transduction of a large variety of cell types, however, the transduction efficiency varies for cell types, and is particularly low for difficult-to-transduce cells such as primary lymphocytes and lymphoma cell lines.

A "target cell" can be any cell that is targeted for transduction with a viral vector. The term "cell" as used in connection with the present invention refers to a single and/or isolated cell, a cell in culture or to a cell that is part of a multicellular entity such as a tissue. In other words, in accordance with the invention, the method is performed *ex vivo or in vitro*. The cell is a vertebrate cell.

Preferably, the cell is a mammalian cell. The term "mammalian cell" as used herein, is well known in the art and refers to any cell belonging to or derived from an animal that belongs to the class of mammalia. Depending on the particular goal to be achieved through modifying the genome of a mammalian cell by transducing it according to the method of the invention, cells of different mammalian subclasses such as prototheria or theria may be used. For example, within the subclass of theria, cells of animals of the infraclass eutheria are preferred, in particular of the order primates, artiodactyla, perissodactyla, rodentia and lagomorpha.

Furthermore, within a species one may choose a cell based on the tissue type and/or capacity to differentiate equally depending on the goal to be achieved by modifying the genome via transducing a target cell according to the ex vivo or in vitro method of the invention. Three basic categories of cells make up the mammalian body: germ cells, somatic cells and stem cells. A germ cell is a cell that gives rise to gametes and thus is continuous through the generations. Stem cells can divide and differentiate into diverse specialized cell types as well as self-renew to produce more stem cells. In mammals, there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell to be used in the method of the invention can be derived from, such as for example hematopoietic stem cells or neuronal stem cells. Human cells are not obtained from a human embryo, in particular not via methods entailing destruction of a human embryo. Human embryonic stem cells are at the skilled person's disposal such as taken from existent embryonic stem cell lines which are commercially available. Accordingly, the present invention to the extent it is implemented with human embryonic stem cells, it does not entail the need to use or destroy a human embryo. Alternatively, or instead of human embryonic stem cells, pluripotent cells that resemble embryonic stem cells such induced pluripotent stem (iPS) cells may be used, the generation of which is state of the art (Hargus et al. 2010; Jaenisch and Young 2008; Takahashi and Yamanaka 2006).

In a preferred embodiment, the target cell is a cell selected from the group consisting of a lymphocyte, a tumor cell, a lymphoid lineage cell, a neuronal cell, an epithelial cell, an endothelial cell, a primary cell, a stem cell.

The term "lymphoid lineage cell" refers to cells that are involved in the generation of lymphocytes and lymphocytes per se. The term "lymphocyte" refers to small lymphocytes (B and T lymphocytes, plasma cells) and natural killer cells as well-known in the art. Lymphoid lineage cells further include, e.g., lymphoid dendritic cells, as well as lymphocyte progenitor cells such as pro-lymphocytes, lymphoblasts, common lymphoid progenitor cells.

A "tumor cell" in accordance with the invention and as is well known in the art is a neoplastic cell involved in the formation of benign, premalignant or malignant tumors. Tumor cells that are malignant are generally referred to as cancer cells and may have the ability to metastasize or spread to neighbouring tissue. Preferred tumor cells, are e. g., pancreatic tumor cells (such as, e.g., AsPC-1 and PANC-1 cells), ovarian cancer lines (such as, e.g. A2780), lymphoma cell lines (such as KARPAS-299, SUDHL-1, SUP-M2, SupT-1, JVM-3, Jeko-1, DoHH-2, HuT-78 and SR-786 cells), skin melanoma cell lines (such as A375) and breast cancer cells (such as, e.g., MCF7, MDA-MB-361 and T47D cells) and primary cells derived directly from tumors.

"Neuronal cells" are well-known in the art and refer to cells that are electrically excitable cells transmitting information by electrical and chemical signalling. Various specialized neuronal cells exist such as, e.g., sensory neurons and motor neurons. For example, basket cells, Betz cells, medium spiny neurons, Purkinje cells, pyramidal cells, Renshaw cells, granule cells or anterior horn cells can be used as target cells in accordance with the invention. A "neuronal tumor cell" is a tumor cell of neuronal origin, for example, Gliomas, Medulloblastoma, Astrocytoma and other cancers derived from neuronal lineage. Glioma cell lines (such as, e.g. U87 and LN18) can be used.

The term "stem cell" is well-known in the art and has been detailed herein above. Preferred stem cells for use according to the method of the invention are, e.g., embryonic stem cells as defined above, induced pluripotent stem cells, hematopoietic stem cells, neuronal stem cells, cancer stem cells, induced pluripotent stem cell (iPS). The present invention does not relate to processes for modifying the germ line genetic identity of human beings.

The term "epithelial cell" is well known in the art. Epithelial cells line cavities and surfaces of structures throughout the body and also form many glands. Epithelial tissues can be classified into simple epithelium (one cell thick) and stratified epithelium (several layers of cells). Epithelial cells are furthermore classified by their morphology into squamous, cuboidal, columnar and pseudostratified epithelial cells. For example, the human stomach and intestine is lined with epithelial cells. Further, epithelial cell lines include also breast carcinoma cells (such as MCF7, MDA-MB-361 and T47D cells) or cells of the cell line HEKT293T.

The term "endothelial cell" is known in the art to refer to cells that line the interior surface of blood vessels and has no other meaning herein. Various different kinds of endothelial cells exist such as, e.g., Ea.hy96, HUVEC and HCAEC cells.

The term "primary cell" as used herein is known in the art to refer to a cell that has been isolated from a tissue and has been established for growth in vitro. Corresponding cells have undergone very few, if any, population doublings and are therefore more representative of the main functional component of the tissue from which they are derived in comparison to continuous cell lines thus representing a more representative model to the *in vivo* state. Methods to obtain samples from various tissues and methods to establish primary cell lines are well-known in the art (see e.g. Jones and Wise, Methods Mol Biol. 1997). Primary cells for use in the method of the invention are derived from, e.g. blood, lymphoma and epithelial tumors. Primary cells also include cells that have been isolated from tissue and immortalized by stable integration of genes into the genome by lentiviral transduction. These genes include CDK-2, CDK4, CDK-6, hTERT, SV40 large T antigen, Bmi1, HPV16 E-6/7 or any combination of two or more of said genes.

With regard to the transduction with viral vectors, particularly with retroviral vectors, primary cells, lymphocytes, lymphoid lineage cells and neuronal cells are considered to be difficult to transduce.

In a more preferred embodiment, the lymphocyte is a primary lymphocyte and/or the tumor cell is a hematopoietic tumor cell, a neuronal tumor cell or an epithelial tumor cell.

Preferably, the epithelial tumor cell is of breast cell tumor origin. It is known in the art that the latter cell as well as the above-mentioned primary cell, lymphocyte and lymphoid lineage cell are particularly difficult to transduce using retroviral vectors and other viral vectors including adenovirus vectors. With the present invention, transduction rates in said cells can be significantly enhanced over state of the art methods.

It is known that cells in culture may be either in suspension or adherent, e.g. adherent to a layer of cells or an artificial substrate.

Said composition in accordance with the first aspect is preferably a solution of said poloxamine and a suspension of said retroviral vector and/or said vertebrate cell.

The present inventors surprisingly discovered that poloxamines are useful as a transduction enhancer for retroviruses. Retroviruses are enveloped viruses and as such very distinct from small molecule drugs or nucleic acids.

Poloxamines exhibit very low or no toxicity; see also the enclosed Examples. In comparison to peptide- and protein-based transduction enhancers, poloxamines can be manufactured at relatively low cost at a GMP quality level for clinical use. Once inside the body, poloxamines are hydrolytically degraded without giving rise to toxic degradation products, usually on a time scale between about two and about five weeks (Cho, Lee, and Webb 2012). These advantageous properties of poloxamines are further illustrated by ophthalmologic applications as described, for example, in Subbaraman et al. 2006 and Tonge et al. 2001.

In a preferred embodiment, (a) said poloxamine is a sequential poloxamine; and/or (b) has a structure of formula (I)

$$R^1R^2N\text{-}(CH_2)_2\text{-}NR^3R^4 \qquad (I)$$

or formula (II)

$$R^1R^2R^5N^+\text{-}(CH_2)_2\text{-}N^+R^3R^4R^6 \; C\text{-}I \qquad (II)$$

wherein in either formula
$R^1$ is $H\text{-}(O(CH_2)_2)_a\text{-} (OCH(CH_3)CH_2)_b\text{-}$;
$R^2$ is $H\text{-} (O(CH_2)_2)_c\text{-} (OCH(CH_3)CH_2)_d\text{-}$ ;

$R^3$ is -$(CH_2CH(CH_3)O)_e$-$((CH_2)_2O)_f$-H;

$R^4$ is -$(CH_2CH(CH_3)O)_g$-$((CH_2)_2O)_h$-H;

$R^5$ and $R^6$ are independently absent or selected from H, C, to $O_6$ alkyl, $C_2$ to $C_6$ alkenyl and $C_2$ to $C_6$ alkinyl; wherein if $R^5$ or $R^6$ are absent, the N bound to $R^5$ or $R^6$ does not carry a positive charge;

C-I stands for one or more counter ions which render the structure of formula (II) electroneutral;

a, c, f and h are independently an integer from about 25 to about 200, preferably from about 50 to about 120; and b, d, e and g are independently an integer from about 5 to about 50, preferably from about 10 to about 25.

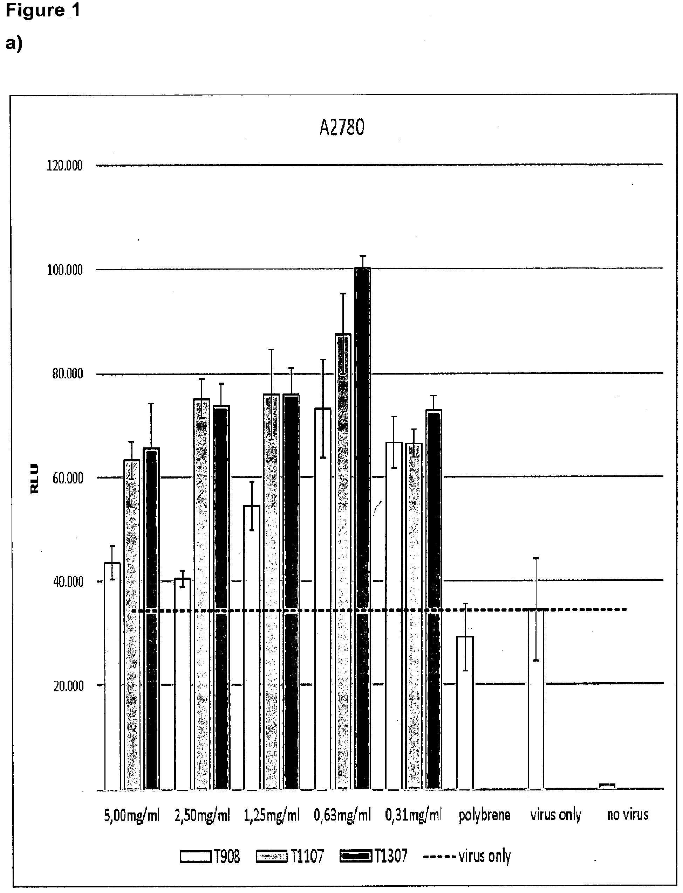

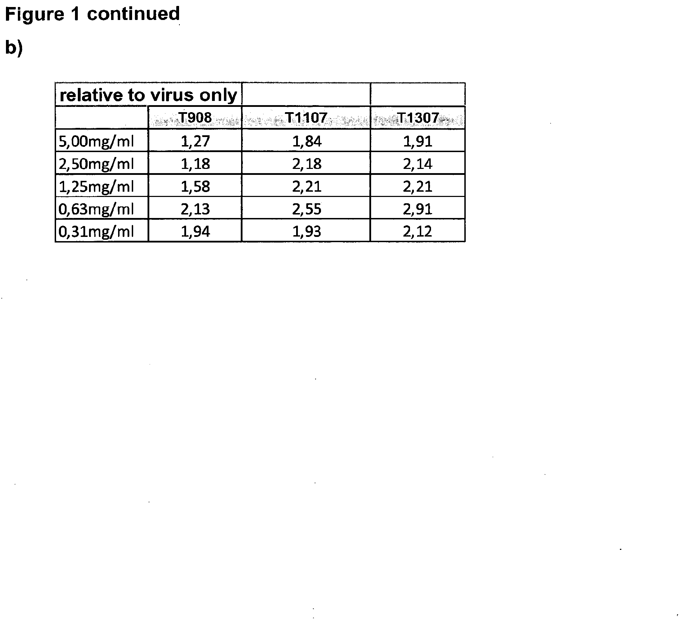

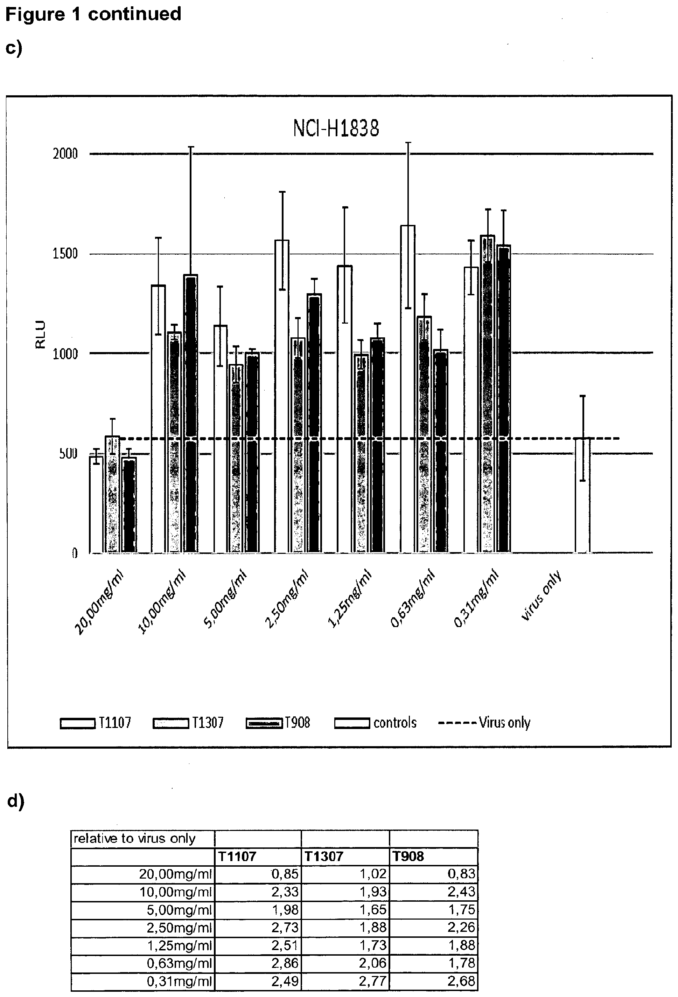

The term "sequential poloxamine" is an art-established term. We refer in this respect to Figure 1 of (Alvarez-Lorenzo et al. 2010). To explain further, sequential poloxamines are characterized in that the polypropylene oxide building blocks are confined to those segments of the four chains bound to the two nitrogens of the central ethylenediamine group, which are directly attached to said nitrogens. Said segments consist of polypropylene oxide building blocks. In other words, in sequential poloxamines the distal segments of the four chains are made of polyethylene oxide.

The reverse applies to reverse sequential poloxamines which are not preferred in accordance with the present invention.

As discussed above, compounds of formula II also embrace compounds of formula (IIa):

$$R^1R^2R^5N^+-(CH_2)_2-NR^3R^4 \ C\text{-}I \qquad (IIa)$$

Preferred counter ions are disclosed further above.

Formulae (I) and (II) as shown in above item (b) depict a sequential poloxamine, wherein the numbers of the building blocks (ethylene oxide (EO) and propylene oxide (PO), respectively) are specified. Preferred PO/EO ratios are disclosed below.

Generally speaking, and throughout this specification, the term "about" refers to an average deviation of +/- 30%, preferably +/- 25%, +/- 20 %, +/- 15 %, +/- 10 % or +/- 5 % from the numeric value which is preceded by the term "about".

Particularly preferred ranges for a, c, f and h are about 27 to about 170, about 32 to about 160, about 38 to about 148, about 40 to about 142, about 43 to about 136, about 46 to about 131, about 49 to about 125, about 51 to about 119, and about 54 to about 114. Particularly preferred ranges for b, d, e and g are about 5 to about 50, about 6 to about 40, about 7 to about 35, about 8 to about 29, about 9 to about 27, about 10 to about 26, about 11 to about 25, about 12 to about 24, about 12 to about 23, and about 11 to about 22.

Especially in relation to the structural parameters defining poloxamines, it is of note that, owing to the method of manufacture, there is a certain variability. In more detail, there is a batch-to-batch variation for a given product, also when obtained from the same manufacturer. In addition, there may be also a variation which is dependent on the manufacturer. This is reflected by the term "about" preceding the structural parameters.

Yet further, within a given batch from a given manufacturer, there is also variability in that distinct molecular species are comprised. For that reason, and if not indicated otherwise, all structural parameters represent averages of the structural parameters of the individual molecular species comprised in a given batch. This is also in line with the information generally provided by manufacturers.

Having said that, the following has to be considered. Owing to the fact that a given sample of a poloxamine contains different molecules or, in other words, exhibits structural heterogeneity, such sample may contain poloxamine molecules which meet the structural requirements of the embodiments disclosed herein and at the same time comprises further poloxamine molecules which do not meet the structural requirements of the embodiments disclosed herein. Even though not preferred, this is a means of implementing the invention. The consequence thereof is as follows: even though the average structural parameter characterizing the poloxamine sample under consideration does not meet the requirements of the embodiments disclosed herein, a fraction of the poloxamine molecules comprised in said sample nevertheless does so. Determining whether such situation occurs can be done by using art-established analytical methods such as mass spectrometry and/or chromatography including gas chromatography. Under such circumstances, the structural parameters recited in the embodiments disclosed herein are parameters characterizing a single poloxamine molecule or the mentioned fraction. It is considered that at least 10%, at least 20%, at least 30%, at least 40% or at least 50% of the poloxamines in a given sample meeting the structural requirements of the embodiments disclosed herein is sufficient to trigger a significant transduction enhancing effect.

In a particularly preferred embodiment of the above disclosed aspects of the invention, a = c; f = h; b = d; and/or e = g; and preferably a = c = f = h; and/or b = d = e = g. Particularly preferred is that a = c = f = h and that b = d = e = g. In other words, it is preferred that $R^1$, $R^2$, $R^3$ and $R^4$ are identical.

In a further particularly preferred embodiment of the above disclosed aspects of the invention, the propylene oxide - ethylene oxide ratio (#PO/#EO) defined as (b + d + e + g) / (a + c + f + h) is in the range from about 0.05 to about 1.5, preferably from about 0.1 to about 1.0, more preferably from about 0.15 to about 0.67, and most preferably from about 0.18 to about 0.35. Preferred are also #PO/#EO ratios of about 0.20, about 0.25 and about 0.30. Corresponding ranges such as from about 0.25 to about 0.35 are also envisaged.

Another common measure is the weight ratio EO/(EO+PO). Preferred poloxamines have an EO/(EO+PO) weight ratio between about 0.3 and about 0.95, more preferably between about 0.5 and about 0.9, and yet more preferably between about 0.7 and about 0.8.

In a preferred embodiment, (a) said poloxamine is in fluid state; and/or (b) said composition is a poloxamine solution in which said vector is dissolved and/or suspended; and/or said cell is in suspension or adherent.

It is known in the art that poloxamines have an upper limit of solubility in water, generally at about 200 mg/ml (Serra-GOmez et al. 2016)). The requirements of this preferred embodiment exclude suspensions of poloxamine, gels as well as any mixtures where dissolved poloxamine would be in an equilibrium with solid poloxamine.

In a further preferred embodiment, said composition furthermore comprises or furthermore consists of (a) one or more polycationic substances selected from the group of polycationic polymers or polycationic peptides; (b) prostaglandins; (c) glycoproteins; (d) poloxamers (e) cyclosporins; and/or (f) staurosporine.

In a second aspect, the present invention provides a pharmaceutical composition comprising or consisting of the composition of the first aspect.

It is understood that pharmaceutical compositions have a beneficial effect. As such, it is envisaged that the viral vector as comprised in said pharmaceutical composition contains a nucleic acid, which is capable of triggering a beneficial effect upon administration to a cell or an organism, said organism including vertebrates, mammals, primates and humans.

In a preferred embodiment of the second aspect, said pharmaceutical composition further comprises or further consists of a pharmaceutically acceptable carrier, excipient and/or diluent. Examples thereof include buffered saline (such as PBS and HBSS), cell culture media (such as DMEM, RPMI1640 and IMDM) and water.

In a third aspect, the present invention provides in vitro or ex vivo use of poloxamine for transducing a vertebrate cell with a retroviral vector, wherein optionally furthermore use is made of

(a) one or more polycationic substances selected from the group of polycationic polymers or polycationic peptides;
(b) prostaglandins;
(c) glycoproteins;
(d) poloxamers; and/or
(e) cyclosporins,

wherein said use does not involve a process for modifying the germ line genetic identity of human beings.

The term "transducing" in the context of cell modulation using viral vectors are well known in the art and has no other meaning herein. Briefly, the term refers to the process of introducing genetic material into a cell and, optionally, its subsequent integration into the genome of said cell via a viral vector. Said genetic material comprises or consists of viral RNA combined with one or more target RNA sequences (hereinafter referred to as target sequences) comprised in said vector intended for integration into (the genome of) a target cell.

Preferred cells as well as preferred enveloped viral vectors are those defined herein above.

According to the invention, (a) uses in the treatment of the human or animal body by therapy are excluded; (b) said use is an in vitro or ex vivo use; and/or (c) said cell is not part of a living organism.

Ex vivo uses are particularly preferred. In other words, cells may be taken from a vertebrate organism, transduced in accordance with the present invention and thereafter re-introduced in a vertebrate organism, preferably but not limited to the organism from which the cells have been taken. The process of transduction occurs outside said organism. Preferably, poloxamine is removed prior to said step of re-introducing.

Generally speaking, in accordance with the invention, poloxamines may be combined with one or more further agents known to enhance transduction (also referred to as "adjuvants" herein).

Accordingly, in a further preferred embodiment, furthermore use is made of (a) one or more polycationic substances selected from the group of polycationic polymers, or polycationic peptides; (b) prostaglandins; (c) glycoproteins; (d) poloxamers; (e) cyclosporins; and/or (f) staurosporine.

"Polycationic polymers" in accordance with the present invention refers to charged polymers whose repeating units bear a positive charge, wherein the positive charge on a repeating unit is stems from protonated nitrogen moieties. For example, in polyethylenimine (PEI) the positively charged group is the imine group.

The term "polycationic peptides" refers to positively charged peptides. For example, poly-L-lysine is a homopolymeric polycationic peptide with the molecular formula of $(C_6H_{12}N_2O)_n$, wherein in accordance with the invention, but without limitation, n may be at least 2, such as at least 20, preferably between 200 and 500, more preferred between 500 and 2500.

In a further preferred embodiment, furthermore use is made of one or more glycoproteins or fragments thereof such as fibronectin and fibronectin fragments (fragments including RetroNectin) as well as protein fragments or peptides selected from the LAH4 peptide family.

The term "glycoprotein" describes proteins, which contain oligosaccharides chains (glycans) covalently attached to one or more amino acid side chains. Fibronectin is a multidomain glycoprotein with a size between 230kDa and 270kDa (Schwarzbauer and DeSimone 2011). Retronectin refers to a fibronectin fragment containing three domains, CS-1, RGDS and heparin-binding domain (Lee et al. 2009). The heparin-binding domain binds to the viral vector and RGDS and CS-1 domains bind to the target cell via VLA-5 and VLA-4 respectively, thus bringing viral particle and cell into close proximity.

The term "LAH4 peptide family" refers to histidine-rich cationic amphipathic peptide (Fenard et al. 2013; Moulay et al. 2017).

In a further preferred embodiment, furthermore use is made of staurosporine.

The term staurosporine refers to a serine/threonine kinase inhibitor originally isolated from the bacterium Streptomyces staurosporeus and has anti-fungal activity (Tamaoki and Nakano 1990). It has also been shown to cause chromatin relaxation in metaphase cells and increase HIV-1 integration in metaphase-arrested cells. Furthermore, staurosporine has been demonstrated to increase the vector copy number (VCN) after lentiviral transduction in human CD34+ peripheral blood cells (Lewis et al. 2018).

In a further preferred embodiment, furthermore use is made of prostaglandins.

The term "prostaglandins" refers to a group of physiologically active lipids which have hormone like effects and mediate pathogenic mechanisms including the inflammatory response. Prostaglandin E2 (PGE2) is primarily produced by macrophages and is involved in the regulation of immune responses, blood pressure, gastrointestinal integrity and fertility (Ricciotti, Emanuela and FitzGerald 2011), In particular, PGE2 has been demonstrated to enhance lentiviral transduction in CD34+ HSPCs (Heffner et al. 2018).

PGE2 is a natural PGE2 receptor agonist. In accordance with the present invention, also other PGE2 receptor agonists may be used. Preferably, the PGE2 receptor agonist is selected from the group consisting of 15d-PGJ2; delta12-PGJ2; 2-hydroxyheptadecatrienoic acid (HHT); Thromboxane A2; Thromboxane B2; Iloprost; Treprostinil; Travoprost; Carboprost tromethamine; Tafluprost; Latanoprost; Bimatoprost; Unoprostone isopropyl; Cloprostenol; Oestrophan; Superphan; Misoprostol; Butaprost; Linoleic Acid; 13(s)-HODE; LY171883; Mead Acid; Eicosatrienoic Acid; Epoxyeicosatrienoic Acid; ONO-259; Cay1039; 16,16-dimethyl $PGE_2$; 19(R)-hydroxy $PGE_2$; 16,16-dimethyl $PGE_2$ p-(p-acetamidobenzamido) phenyl ester; 11-deoxy-16,16-dimethyl $PGE_2$; 9-deoxy-9-methylene-16,16-dimethyl PGE2; 9-deoxy-9-methylene $PGE_2$; Sulprostone; $PGE_2$ serinol amide; $PGE_2$ methyl ester; 16-phenyl tetranor $PGE_2$; 15(S)-15-methyl $PGE_2$; and 15 (R)-15-methyl $PGE_2$.

More generally speaking, agents which stimulate prostaglandin E receptor signaling may be used. Such agents include $PGA_2$; $PGB_2$; $PGD_2$; $PGE_1$ (Alprostadil); $PGF_2$; $PGI_2$ (Epoprostenol); $PGH_2$; and $PGJ_2$.

Further agents known to stimulate the prostaglandin signaling pathway are: Mebeverine, Flurandrenolide, Atenolol, Pindolol, Gaboxadol, Kynurenic Acid, Hydralazine, Thiabendazole, Bicuclline, Vesamicol, Peruvoside, Imipramine, Chlorpropamide, 1,5-Pentamethylenetetrazole, 4-Aminopyridine, Diazoxide, Benfotiamine, 12-Methoxydodecenoic acid, N-Formyl-Met-Leu- Phe, Gallamine, IAA 94 and Chlorotrianisene.

In accordance with the invention, one or more said polycationic substances may be brought into contact with the target cell. Said contacting can be effected prior to, concomitant with or after the target cell has been brought into, contact with the viral vector and said poloxamine, as long as all of the latter components are eventually present at the same time to simultaneously contact the target cell.

If more than one polycationic substance such as at least two, at least three, at least four, at least 5 or at least 6 polycationic substances are further brought into contact with the target cell, they may belong to either polycationic polymers or to polycationic peptides, or they may be chosen from both, i.e. polycationic polymers can be mixed with polycationic peptides.

Without being bound to a specific theory, it is suggested that all of the polycationic substances mentioned herein are capable to bridge electrostatic repulsion between the retroviral vectors and target cells and, thus, can further enhance transduction efficiency.

In a more preferred embodiment, said polycationic polymers are selected from the group consisting of poly(ethylene glycol)-poly(L-lysine) block copolymer (PEG-PLL) and 1,5-Dimethyl-1,5-Diaza-undeca-methyl-polymethobromide (Polybrene); and/or said polycationic peptides are selected from the group consisting of protamine sulfate and poly-l-lysin (PLL) having a mean molecular weight from about 1 to about 300 kDa. Further envisaged are PGE-2, retronectin, poloxamers and other transduction enhancing substances.

Poloxamers are copolymers comprising a central polypropylene oxide chain flanked by two polyoxyethylene chains. Several trade names are in use for poloxamers such as Synperonic®, Pluronic® and Kolliphor®. Frequently, poloxamers are characterized by a numerical code. To give an example, poloxamer 407 is a poloxamer with a polyoxypropylen part having a molecular mass of 4000 g/mol and 70% polyoxyethylene content. Poloxamers, including poloxamers particularly suitable for transduction, are described in the present applicant's earlier application WO 2013/127964. Particularly preferred poloxamers are disclosed further below.

Protamines are a group of small highly basic arginine-rich proteins. They are found to be associated with DNA in sperm in many species. Protamine sulfate consists of sulfates of these basic peptides extracted from the sperm or roe of fish. Generally, prolamines are mixtures of several similar peptides. Typical lengths are about 30 amino acids. The average molecular mass is between 4000 and 7000, preferably between 5000 and 5500 Da.

Protamine sulfates are described, for example, in the assessment report for protamine containing medicinal products EMA/741250/2012 of the European Medicines Agency.

The term "poly(ethylene glycol)-poly(L-lysine) block copolymer (PEG-PLL)" refers to positively charged macromolecules with the molecular formula of $[C_6H_{12}N_2O]_x$-$[C_2H_4O]_yH_2O$, wherein preferably x is about 48 and y is about 272,72 corresponding to an average molecular weight for poly(ethyleneglycol) (PEG) of about 12000 Da. PEG-PLL is synthesized as described by (Harada and Kataoka 1995).

PEG-PLL can be used alone or, in combination with any of polybrene, protamine sulfate and/or poly-L-lysin. Conceivably, any polycationic substance will be used at concentrations essentially not affecting cell viability. For example, the generally accepted working concentration for polybrene is 8 to 10 μg/ml.

In an even more preferred embodiment, the polycationic substances are 1,5-dimethyl-1,5-diaza-undeca-methyl-polymethobromide and/or protamine sulfate.

1,5-dimethyl-1,5-diaza-undeca-methyl-polymethobromide and protamine sulfate can be used alone or in combination with each other and/or further polycationic substances.

In accordance with the invention, one or more glycoproteins or peptides from the LAH4 peptide family, and/or prostaglandins and or may be brought into contact with the target cell. Said contacting can be effected prior to, concomitant with or after the target cell has been brought into contact with the viral vector and said poloxamine, as long as all of the latter components are eventually present at the same time to simultaneously contact the target cell.

One, more or all of the above disclosed additional agents (i.e., in addition to at least one poloxamine) may be used.

We note that for poloxamines with a certain propylene oxide-ethylene oxide ratio (#PO/#EO), a synergistic effect of the combined use of poloxamine with protamine sulfate is observed. We refer in this respect to the experimental data comprised herein. The term "propylene oxide-ethylene oxide ratio" is defined herein below. Preferred ranges of this ratio in relation to the mentioned synergistic effects is between about 0.18 and about 0.35.

Cyclosporins are a group of macrolides found in fungi which find application as immunosuppressants. In accordance with the present invention, they may be used for the enhancement of transduction in conjunction with poloxamines and optionally further agents which enhance the transduction as described herein.

In a fourth aspect, the present invention provides an in vitro or ex vivo method of transducing a vertebrate cell with a retroviral vector, said method comprising or consisting of bringing into contact said vertebrate cell, a poloxamine, and said viral vector, wherein preferably said bringing into contact is effected in the following order: (a) providing said cell; (b) adding said poloxamine; and (c) adding said vector, wherein preferably after said bringing into contact spinoculation is performed, wherein said method is not a process for modifying the germ line genetic identity of human beings.

Related thereto, the present invention provides a method of transducing a vertebrate cell with a lentiviral vector, said method comprising or consisting of bringing into contact said vertebrate cell, a poloxamine, and said viral vector, wherein preferably said bringing into contact is effected in the following order: (a) providing said cell; (b) adding said poloxamine; and (c) adding said vector, wherein preferably after said bringing into contact spinoculation is performed.

Steps (b) and (c) may also be performed concomitantly.

The terms "contacting" and "bringing into contact" refer to mixing a target cell with a viral vector and a poloxamine such that the transduction event can occur. Conditions for contacting that allow the transduction event to occur are well known in the art and may depend to a certain extent on the target cells and the viral vector chosen. For example, some target cells are more difficult to transduce than other cells and may need to be transitioned into a specific culture medium before transduction with a viral vector can be achieved. Corresponding methods and conditions are described for example in (Chu, Cornetta, and Econs 2008; Jacome et al. 2009; Poczobutt et al. 2010) Further exemplary conditions are described in the example section. The retroviral vector and the poloxamine can be added simultaneously, e.g. as a mixture, to the target cells or in sequential mode, as long as both compounds are simultaneously in contact with the target cell to allow transduction. Preferably, the target cell, viral vector and poloxamine are contacted for at least 5 hours, such as at least 6, at least 7, at least 8, more preferred at least 9, at least 10, at least 11, and most preferred at least 12 hours. Also envisaged are longer contacting times such as at least 13, at least 14, at least 15, at least 16, at least 24, at least 36, at least 48 or at least 72 hours. Preferred is between about 24 and about 72 hours.

To the extent one or more of the transduction adjuvants disclosed herein is to be used, it is preferred that said adjuvant is added in step (b); and/or after step (a) and prior to step (c).

Preferred embodiments of the use of the third aspect define preferred embodiments of the in vitro or ex vivo method of the fourth aspect.

According to the invention, (a) methods of treatment of the human or animal body by therapy are excluded; (b) said method is an in vitro or ex vivo method; and/or (c) said cell is not part of a living organism.

In a further preferred embodiment of said method, and in analogy to a preferred embodiment of the use in accordance with the present invention, said bringing into contact comprises or further consists of bringing into contact with (a) one or more polycationic substances selected from the group of polycationic polymers or polycationic peptides; (b) prostaglandins; (c) glycoproteins; (d) poloxamers; (e) cyclosporins; and/or (f) staurosporine.

Preferred polycationic polymers, polycationic peptides, glycoproteins, prostaglandins and poloxamers are those disclosed herein above.

In another preferred embodiment, the above disclosed method comprises the further step of spinoculating said viral vector with said target cell concomitant with or after contacting said target cell with said poloxamine.

The term "spinoculating" relates to centrifugal inoculation of a target cell with the viral vector to ensure close contact for cellular uptake of retroviruses. Spinoculation protocols are well known in the art and described in the literature discussed in the introductory part of this document. A spinoculation step can be executed, concomitant with or after contacting said target cell with said poloxamine. Preferably, the spinoculation step is performed after contacting the target cell with the poloxamine and the viral vector.

The spinoculation step further increases transduction rates achieved with the method of the invention, particularly in cells that are difficult to transduce.

Preferred in conjunction with spinoculation, but not necessarily only in conjunction with spinoculation, is the use of VSV-pseudotyped retroviral vectors and VSV-pseudotyped lentiviral vectors. VSV-pseudotyping is a means of conferring inter alga mechanical stability to vectors. Such mechanical stability is advantageous in the context of spinoculation. VSV-pseudotyped vectors are described in applicant's patent application WO 2015/104376. Further disclosure in relation to VSV-pseudotyped is provided herein further above.

In a preferred embodiment, transduction is enhanced as compared to a reference method, which reference method is without adding said poloxamine, said reference method being otherwise identical to said method of transducing a vertebrate cell.

Transduction and enhancement of transduction may be quantified by art-established methods. As explained in the examples, this can be done, for example, by fluorescence-activated cell sorting (FACS). FACS allows to determine the number of transduced cells. A cell is classified as being either transduced or not transduced on the basis of whether a fluorescent marker is present or not. Transduction may be expressed in relative terms, for example as the ratio of the number of transduced cells divided by the total number of cells used in the given transduction assay.

In a fifth aspect, the present invention provides the composition of the first aspect, wherein said composition of the first aspect comprises said viral vector, or a pharmaceutical composition of the second aspect for use in medicine.

In a sixth aspect, the present invention provides the pharmaceutical composition of the second aspect or the composition for use of the fourth aspect, wherein said retroviral vector carries a gene which is beneficial for an individual suffering from a disease or being at risk of developing said disease. Preferred diseases and associated genes, respectively, are given in Table 1 below.

**Table 1**

| Disease | Gene(s) |
|---|---|
| Cystic fibrosis | CFTR |
| Sickle cell anemia | haemoglobin A |
| B-thalassemia | HBB |
| Huntington | HTT |
| Gaucher disease | Glucocerebrosidase |
| Fanconi anemia | FANCA, FANCB, FANCC, FANCD1 (BRCA2), FANCD2, FANCE, FANCF, FANCG, FANCI, FANCJ (BRIP1), FANCL, FANCM, FANCN (PALB2), FANCP (SLX4), FANCS (BRCA1), RAD51C and XPF |
| Haemophilia | Factor VIII, Factor IX |
| Duchenne muscular dystrophy | DMD |
| PKD | PKD1, PKD2, and PKHD1 |
| SCID | IL2RG |
| Cancer | p53 |
| Neurodegenerative diseases | AD, SCA1, APP, PSEN1+2, SCNA, LRRK1 |
| Familial Dysalbuminemic Hyperthyroxinemia | Albumin |

In a further preferred embodiment, said retroviral vector is a lentiviral vector.

In a further preferred embodiment of the above disclosed aspects of the present invention, said poloxamine has a molecular weight from about 5 to about 50 kDa, preferably from about 10 to about 40 kDa or from about 10 to about 30 kDa, and more preferably from about 15 kDa to about 30 kDa or from about 15 to about 25 kDa.

Preferred poloxamines are T1504, T1304, T1104, T904, T704, T504, T304, T1307, T1107, T707, T1508 and T908. Particularly preferred are poloxamines T1307, T1107, T707, T1508 and T908. Especially preferred are the poloxamines selected from T908, T1107 and T1307. Most preferred is T1107.

The last digit (e.g. "8" in case of T908) indicates the value of the weight ratio (EO/(EO+PO)). In case of the last digit being 8, said ratio is about 0.8.

The number of propylene oxide (PO) building blocks, the number of ethylene oxide (EO) building blocks and the molecular weight of these three particularly preferred poloxamines is given in Table 2 below.

**Table 2**

|  | **#PO** | **#EO** | **MW (g/mol)** |
|---|---|---|---|
| T908 | about 86,2 | about 454,5 | about 25000 |
| T1107 | about 77,6 | about 238,6 | about 15000 |
| T1307 | about 93,1 | about 286,4 | about 18000 |

The "T"-designations are established in the art. We refer in this respect to (Chiappetta and Sosnik 2007; Schmolka 1977).

The letter "T" is derived from the term "Tetronic®" which is frequently used in the art to designate commercially available poloxamines. Poloxamines are available from several manufacturers including BASF and Croda.

In a preferred embodiment of any of the preceding aspects of this invention, said poloxamine is provided in a concentration in the range from about 0.1 to about 40 mg/ml, preferably from about 0.2 to about 30 mg/ml, about 0.25 to about 25 mg/ml, more preferably between about 0.3 and about 20 mg/ml, wherein (a) if said cell is a cell in suspension, about 0.3 to about 20 mg/ml or about 5 to about 20 mg/ml is preferred, about 1 to about 20 mg/ml or about 10 to about 20 mg/ml being more preferred; and (b) if said cell is adherent, about 0.3 to about 10 mg/ml, about 0.3 to about 5 mg/ml, about 0.3 to about 2 mg/ml, about 0.3 to about 1.25 mg/ml, and about 0.3 to about 1 mg/ml are increasingly preferred. Especially preferred are concentrations between about 0.6 and about 1 mg/ml such as about 0.63 mg/ml.

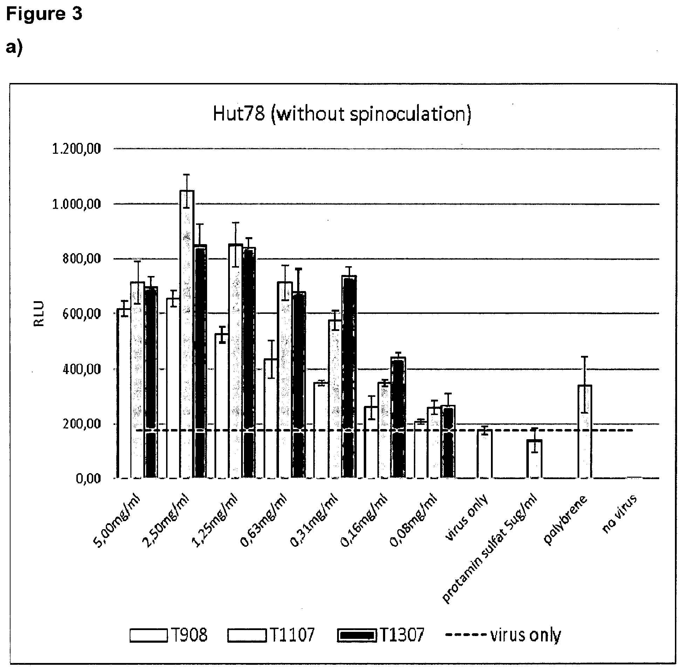

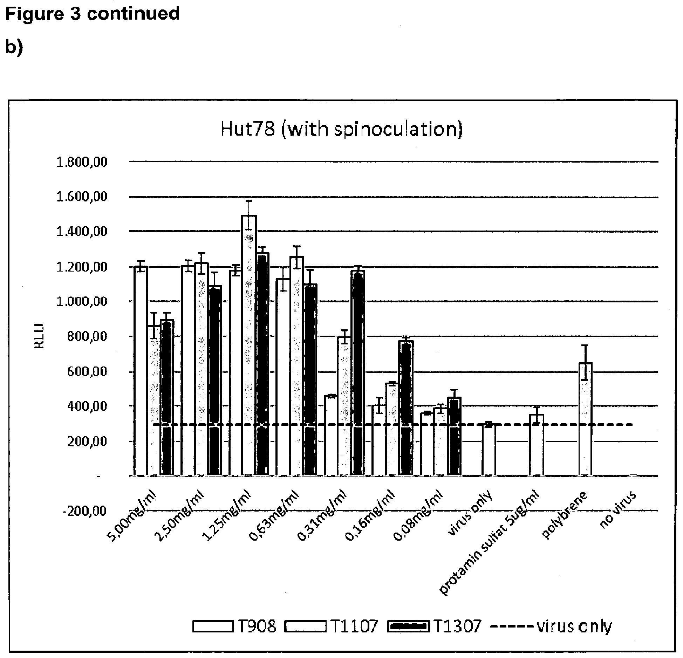

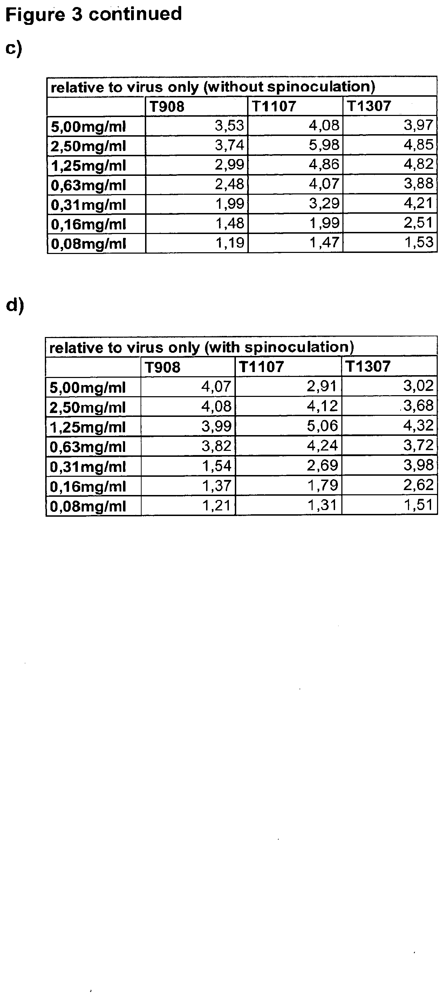

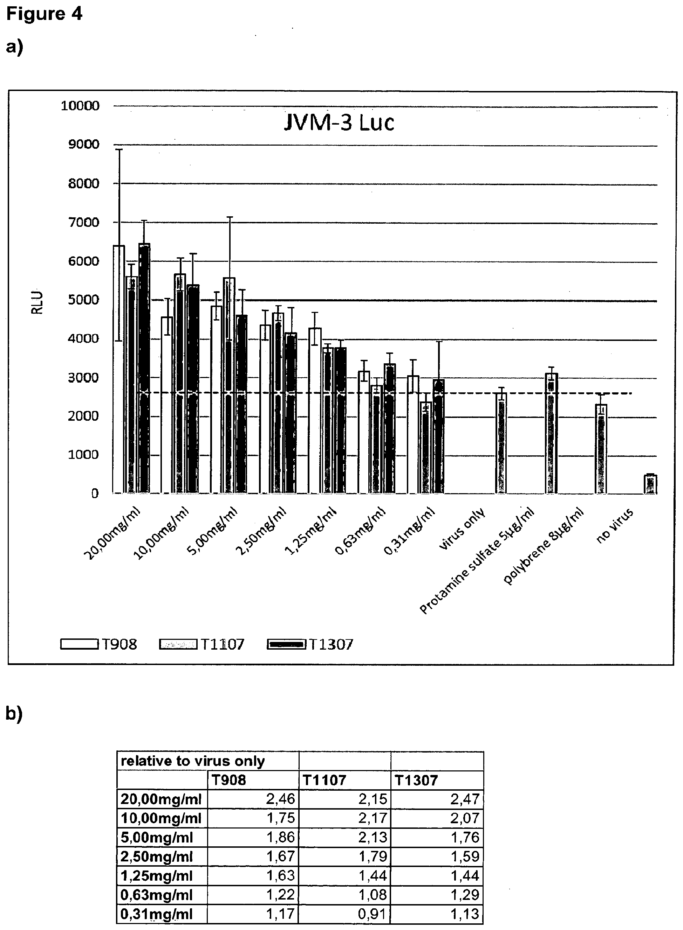

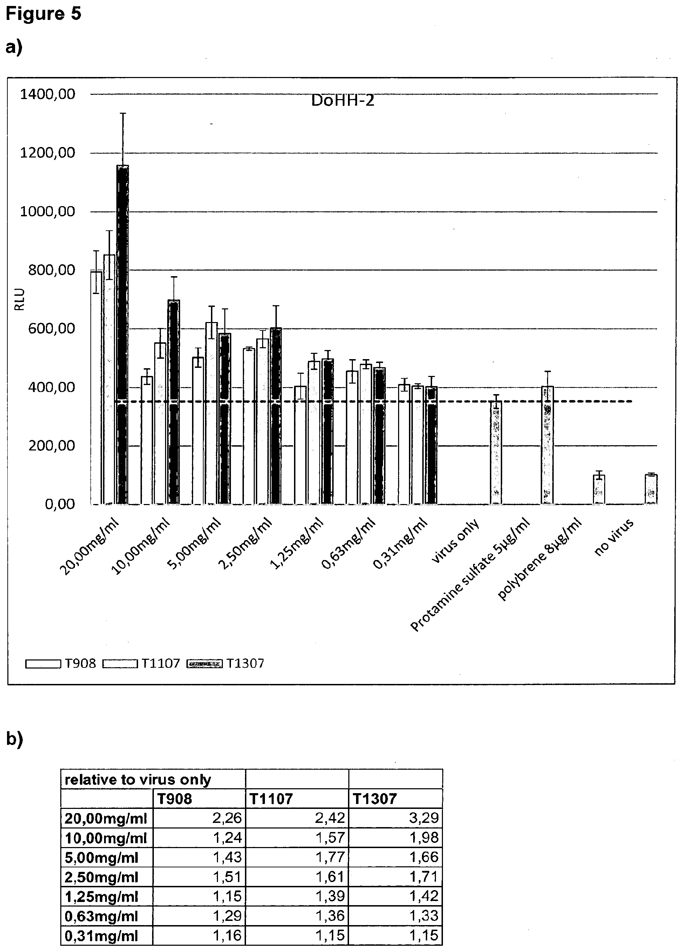

Examples of cells in suspension are JVM-3 and DoHH-2 cells; for data see Figures 4 and 5. Examples of adherent cells are A2780 cells; see Figure 1. Hut78 cells (cells in suspension) prefer concentrations between 0.5 and 5 mg/ml; see Figure 3.

Poloxamines as defined herein are preferably dissolved in water, phosphate buffer or directly in cell culture medium. Poloxamines can be dissolved, e.g., in water or phosphate buffer to obtain e.g. 100 mg/ml or 50 mg/ml stock solutions that can be diluted to a given working concentration. At concentrations of more than 200 mg/ml poloxamine solutions are usually gel-like. At concentrations below 200 mg/ml poloxamine solutions are in a fluid state. Preferably, the concentrations are such that the poloxamine is provided in a fluid state or in solution.

In a more preferred embodiment, said poloxamine is provided at a concentration of about 500 to about 1000 µg/ml.

Poloxamines as defined herein are in a fluid state when diluted in water or phosphate buffer. As will be understood by the skilled person, transducing cells with fluid poloxamines may be more convenient as, e.g., it allows convenient handling such as easier pipetting.

In a preferred embodiment of the composition or the composition for use in accordance with the present invention, said composition comprises or further consists of (a) one or more polycationic substances selected from the group of polycationic polymers or polycationic peptides; (b) protaglandins; (c) glycoproteins; (d) poloxamers; (e) cyclosporins; and/or (f) staurosporine.

Preferred polycationic polymers or polycationic peptides are those disclosed herein above.

In a further preferred embodiment, said poloxamine is in fluid state and/or in solution.

In a further preferred embodiment of any of the preceding aspects, said vertebrate cell is a mammalian cell, preferably a primate cell or human cell.

In a preferred embodiment of any of the preceding aspects, transduction efficiency is increased (a) in a statistically significant manner; and/or (b) at least 1.25-fold, at least 1.5-fold, at least 1.75-fold, at least 2-fold or at least 5-fold as compared to transduction in the absence of said poloxamine. It is of note that for large scale production of genetically corrected patient cells, such increases are very significant.

The transduction efficiency for a given experiment is defined as the proportion of cells transduced by the vector, or by the total level of transgene expression in the target cells. Poloxamines provide for the recited increases of said proportion or said transgene levels compared to cells transduced without transduction enhancer. Exemplary assays for determining transduction efficiency are described in Example 1. Evidence for the enhancement of transduction can be found in the Examples enclosed herewith.

Generally speaking, it is particularly preferred that (a) said polycationic polymers are selected from 1,5-dimethyl-1,5-diaza-undeca-methyl-polymethobromide (Polybrene) and poly(ethylene glycol)-poly(L-lysine) block copolymer (PEG-PLL); (b) said polycationic peptides are selected from protamine sulfate, poly-L-lysin (PLL) having a mean molecular weight from about 1 to about 300 kDa, more preferably between about 1 kDa and about 100 kDa, and Vectofusin-1®; (c) said glycoprotein is fibronectin (RetroNectin®); (d) said prostaglandine is PGE2; (e) said poloxamer is Synperonic® F108 or Synperonic® F98 (see also WO 2013/127964); and/or (f) said cyclosporine is CsA or CsH.

A preferred prostaglandin is prostaglandin-E2 (PGE2) (Heffner et al. 2018). A preferred glycoprotein is Retro-Nectin (Murray et al. 1999).

A further useful poloxamer is poloxamer 407.

In a particularly preferred embodiment, protamine sulfate is the polycationic peptide; #PO/ #EO is from about 0.18 to about 0.35; and/or said poloxamine is T1107, T1307 or T908.

The latter particularly preferred embodiment is characterized by a surprising synergistic effect conferred by the combined use of protamine sulfate and poloxamines meeting the recited propylene oxide-ethylene oxide ratio. Evidence is comprised in the Examples.

The present disclosure furthermore relates to a kit comprising or consisting of (a) a poloxamine, preferably as defined herein; (b) optionally one, more or all of the following (i) to (iii): (i) one, more or all of (1) to (3): (1) polycationic substances selected from polycationic polymers and polycationic peptides; (2) prostaglandins; (3) glycoproteins; (4) poloxamers; and (5) cyclosporins; (ii) retroviral vector; and (iii) a vertebrate cell; and (c) optionally a manual comprising instructions for performing the uses of and/or the methods of the invention.

The kit of the present disclosure comprises instructions for use, preferably in accordance with the uses or methods disclosed above.

The definitions and combinations of features described herein above with regard to the polycationic polymer, the polycationic peptide, the glycoprotein, the prostaglandin, and the poloxamer apply mutatis mutandis also to the kit of the present disclosure.

Said kit of the present disclosure comprises or consists of said poloxamine and said retroviral vector.

Said kit of the present disclosure comprises or consists of said poloxamine and said vertebrate cell.

Said kit of the present disclosure comprises or consists of said poloxamine, said retroviral vector and said vertebrate cell.

The various components of the kit of the present disclosure may be packaged in one or more containers such as one or more vials. The vials may, in addition to the recited components, comprise preservatives or buffers for storage, media for maintenance and storage, e.g. cell culture media, DMEM, MEM, HBSS, PBS, HEPES, hygromycin, puromycin, Penicillin-Streptomycin solution, gentamicin inter alia. Advantageously, the kit comprises instructions for use of the components allowing the skilled person to conveniently work, e.g., various embodiments of the invention. Any of the components may be employed in an experimental setting.

In a further aspect, the present invention provides a method of enhancing viral copy number (VCN), said method comprising the method of transducing of the fourth aspect, wherein VCN is enhanced as compared to a reference method, which reference method is without adding said poloxamine, said reference method being otherwise identical to said method of enhancing VCN, wherein preferably the enhancement of VCN is at least 1.5-fold, at least 2-fold, at least 3-fold, at least 5-fold or at least 10-fold.

In other words, provided is a method of enhancing viral copy number (VCN), said method comprising bringing into contact a vertebrate cell, a poloxamine, and a viral vector, wherein preferably said bringing into contact is effected in the following order: (a) providing said cell; (b) adding said poloxamine; and (c) adding said vector, wherein preferably after said bringing into contact spinoculation is performed, and wherein VCN is enhanced as compared to a reference method, which reference method is without adding said poloxamine, said reference method being otherwise identical to said method of enhancing VCN.

In a preferred embodiment of the above disclosed method of enhancing VCN and furthermore of the method of the fourth aspect, (a) said retroviral vector is a lentiviral vector; and/or (b) said poloxamine is T1107.

The term "vector copy number" has its art-established meaning. It refers to the number of copies of a vector or portion thereof in a cell, preferably in the cell's genome. The vector copy number may be determined for individual cells, and also for a population of cells. In the latter case, an average VCN is obtained. Methods for determining the vector copy number are known in the art. They include quantitative polymerase chain reaction (qPCR) and digital droplet polymerase chain reaction (ddPCR) as described, e.g., in Hauber et al. 2018 (Lin et al. 2016).

Control and improvement of the VCN is an important aspect in gene therapy applications. The VCN determines the amount of therapeutic gene expressed in a cell, which directly affects therapeutic success. Increasing VCN by using transduction enhancers allows the reduction of lentiviral vector used and thus reduces potential risk and also costs of treatment.

Experimental evidence of the enhancement of VCN when using the methods of the present invention can be found in the enclosed examples and figures.

The definitions and combinations of features described herein above with regard to the viral vector and poloxamine apply mutatis mutandis also to the viral vector and poloxamine of the kit of the invention. For example, the retroviral vector can be a lentiviral vector that may or may not be further modified such as, e.g., pseudotyped.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The figures show:

**Figure 1:** (a) Luminescene signal of A2780 cells transduced with a Lentivirus expressing Luciferase. Transduction of A2780 cells in triplicate with a lentivirus expressing Luciferase. Three different Poloxamines (T908, T1107, T1307) at concentrations between 0.31 mg/ml to 5 mg/ml were used. The diagram shows relative light units (RLU) measured 3d after transduction with a plate reader. As controls, polybrene (8 $\mu$g/ml) and virus only (without adjuvant) were used. Dotted line indicates virus only Luciferase level.
(b) Fold improvement of transduction efficiency in A2780 compared to virus only based on the luciferase signal of transduced cells.
(c) Luminescence signal of NCI-H1838 cells transduced with a Lentivirus expressing Luciferase. Transduction of A2780 cells in triplicate with a lentivirus expressing Luciferase. All conditions are the same as described in Fig. 1 a
(d) Fold improvement of transduction efficiency in NCI-H1383 compared to virus only based on the luciferase signal of transduced cells.

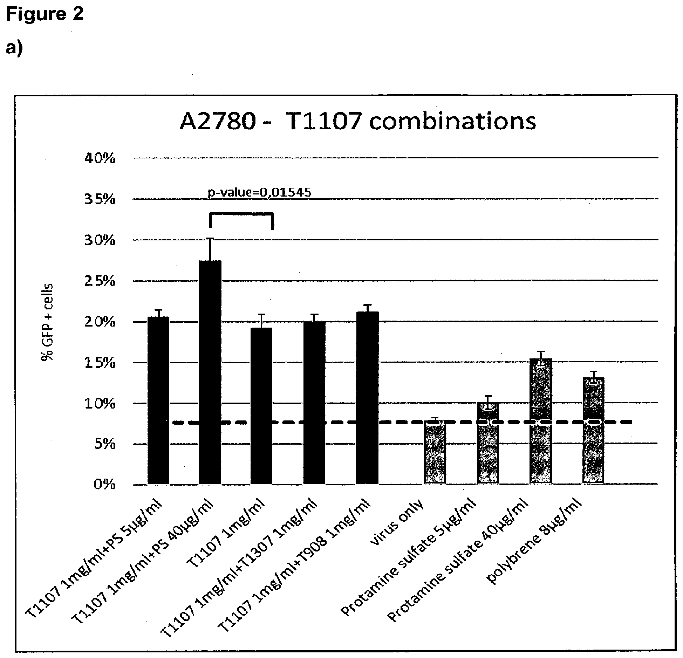

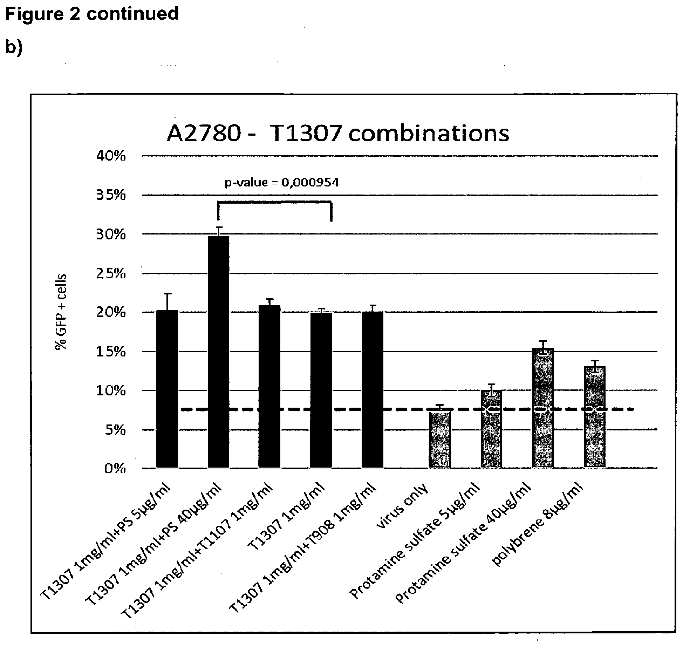

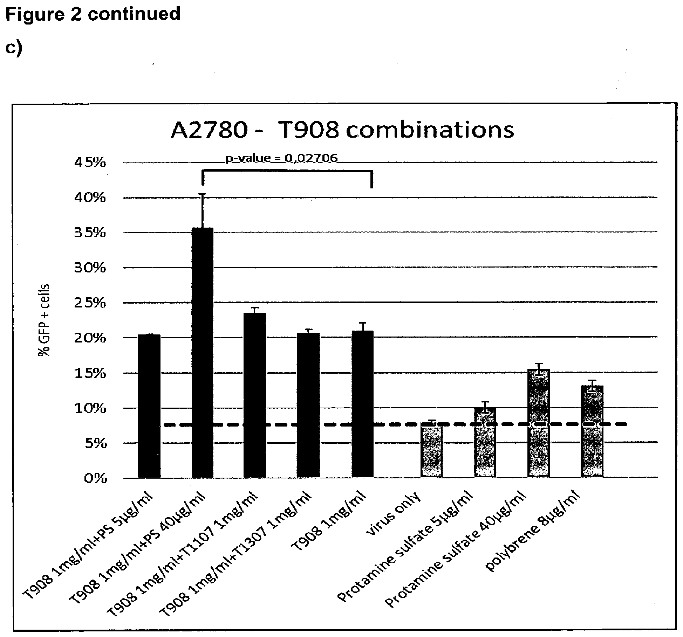

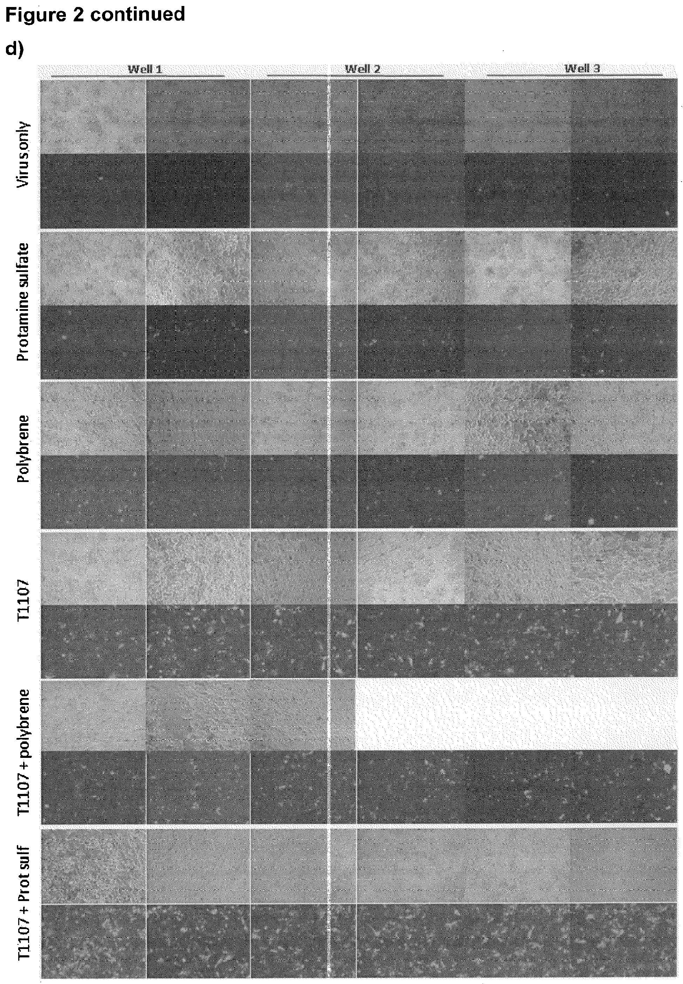

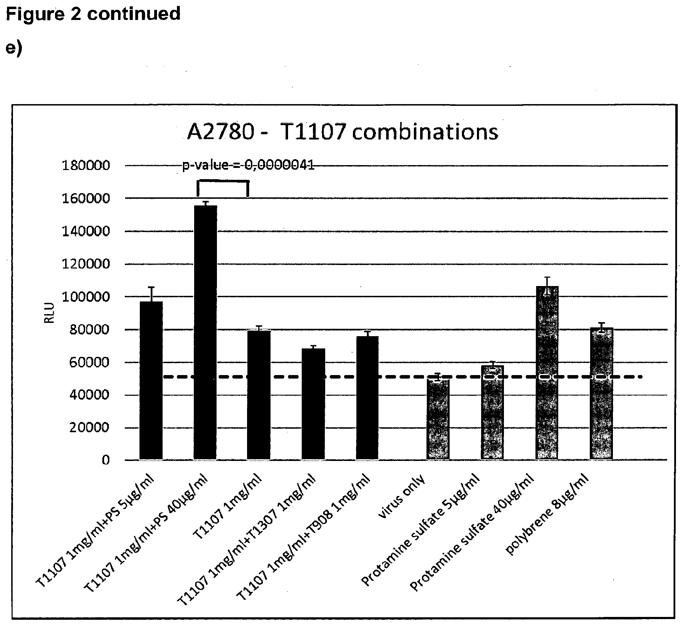

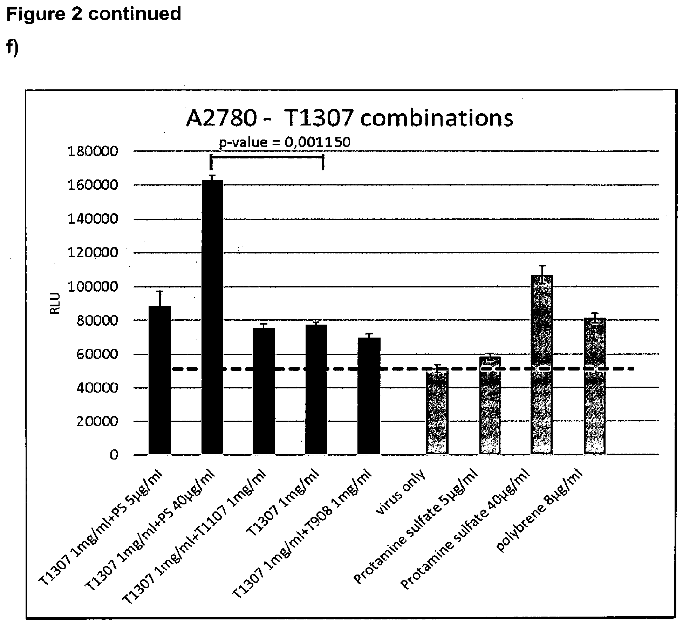

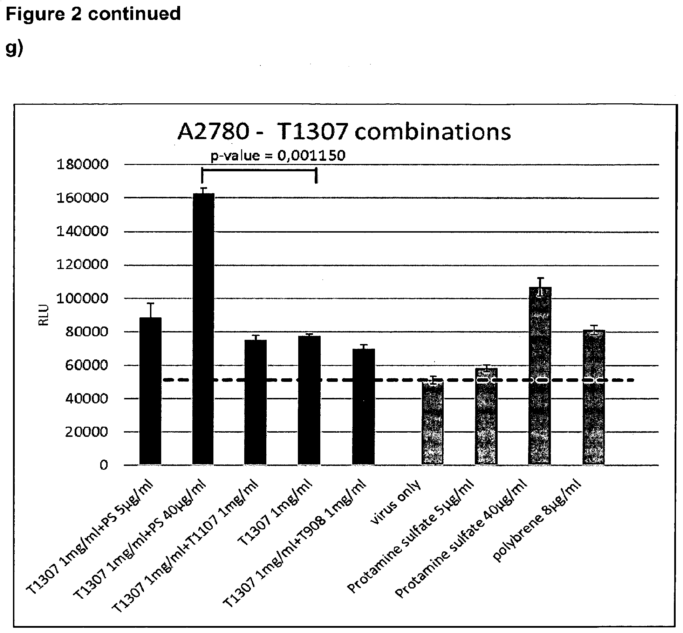

**Figure 2:** Additive effects of Poloxamines and other transduction enhancing substances. 1mg/ml of each Poloxamines T1107, T1307 and T908 was combined with 5 $\mu$g/l or 40 $\mu$g/ml Protamine sulfate (PS) or 1 mg/ml of a corresponding other poloxamine. Efficiency of transduction enhancement on a cellular basis was measured by transduction with a GFP expressing virus and counting of GFP expressing cells compared to total cells 3 days after transduction (Fig. 2 a-c). Example images from 3 different wells per condition of virus only, 8 $\mu$g/ml Polybrene, 40 $\mu$g/ml Protamine sulfate, 1 mg/ml T1107, 1 mg/ml T1107 with 8 $\mu$/ml Polybrene and1 mg/ml T1107 with 40 $\mu$g/ml Protamine sulfate and are shown in Fig. 2 d. To determine total transduction efficiency cells were transduced with a lentivirus expressing Luciferase. Luminescence was measured 3 days after transduction and is shown in relative light units (RLU; Fig. 2 e - g). Significance of differences between Poloxamine only and Poloxamine with 40 $\mu$g/ml Protamine sulfate was calculated by a 2-tailed student's t-test and shown as p-value. Dotted lines in Graphs indicate level of virus only control.

**Figure 3:** Luminescence level in relative light units (RLU) of Hut78 suspension cells transduced with Lentivirus expressing Luciferase without a) and c) and with b) and d) spinoculation. Tetronics and controls were added at the indicated concentrations. (c) and (d) show the corresponding fold-improvements of transduction over virus only control.

**Figure 4:** JVM-3 suspension cells were transduced without spinoculation with Luciferase expressing Lentivirus. a) shows Luminescence level in relative light units (RLU) measured 3 days post transduction. Tetronics and controls were added at the indicated concentrations. (b) shows the corresponding fold-improvements over virus only control.

**Figure 5:** DoHH-2 suspension cells were transduced without spinoculation with Luciferase expressing Lentivirus. a) shows luminescence level in relative light units (RLU) measured 3 days post transduction. Tetronics and controls were added at the indicated concentrations. (b) shows the corresponding fold-improvements over virus only control.

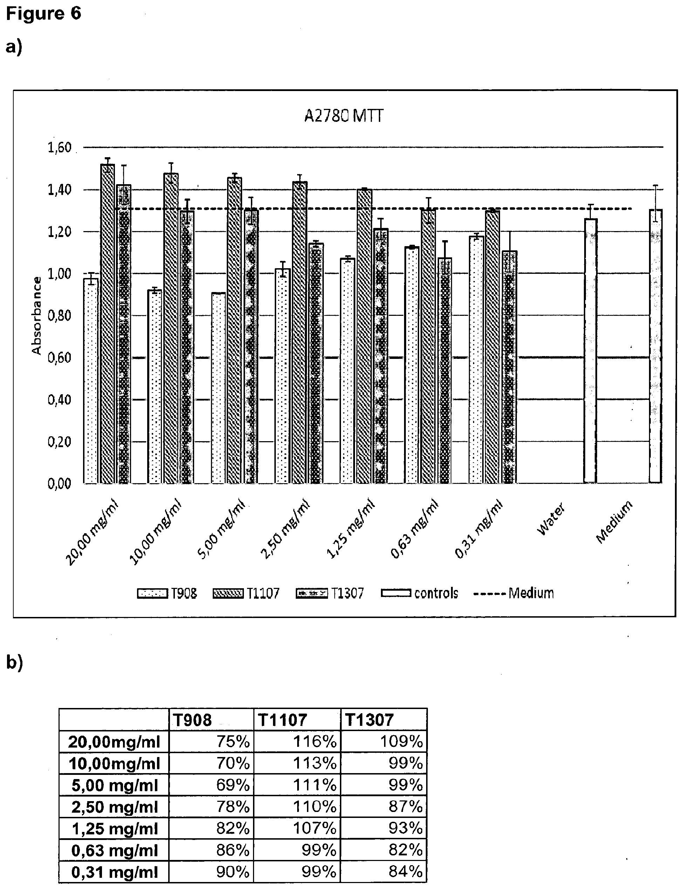

**Figure 6:** Viability of A2780 cells treated with various concentrations of Poloxamines measured by MTT colorimetric assay. Graph a) shows mean values of measured absorbance, bars indicate standard deviations. Dotted line in graph indicates medium control. %-values in b) are relative viability compared to medium alone.

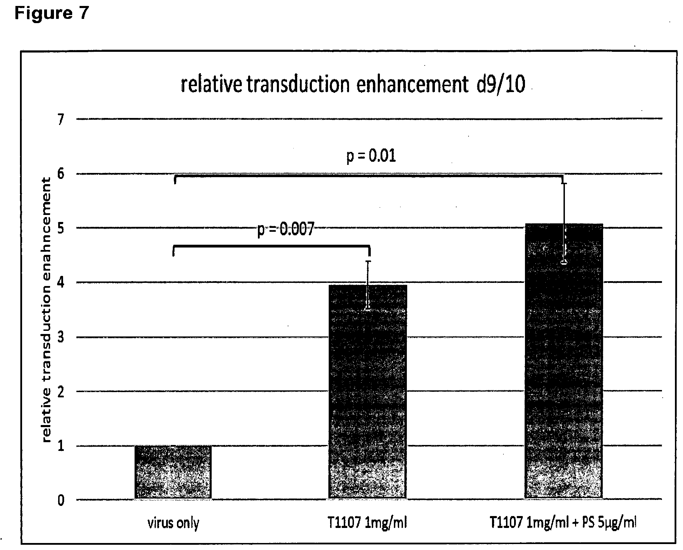

**Figure 7:** Relative transduction enhancement using 1 mg/ml T1107 either alone or in combination with 5 µg/ml PS compared to transduction with virus only. Transduction enhancement was measured by FACS analysis on day 9/10 post transduction in PBMCs from independent donors. Bars show mean values from 3 different donors. The percentage of GFP+ cells transduced with transduction enhancer was compared to cells transduced with virus only. This ratio gives the relative transduction enhancement. Error bars show standard deviations. P-values are calculated with a 2-tailed student's t-test.

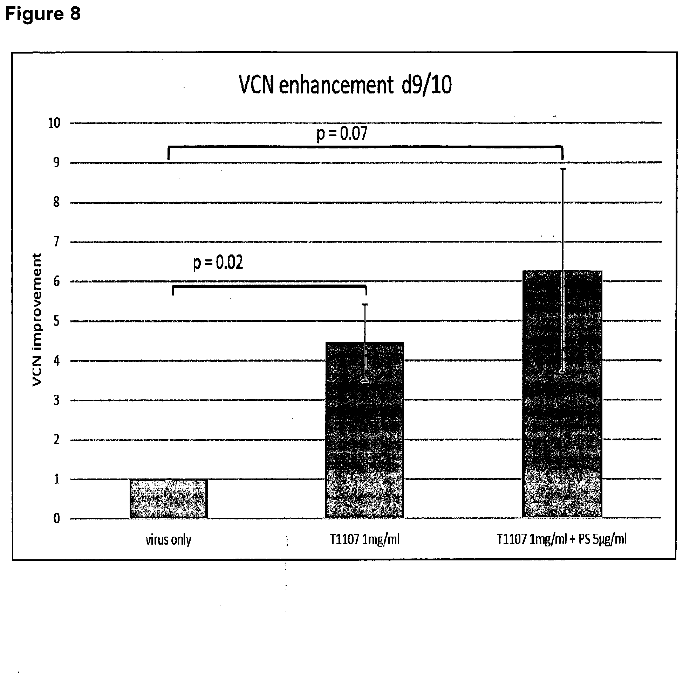

**Figure 8:** Relative vector copy number (VCN) improvement using 1 mg/ml T1107 either alone or in combination with 5 µg/ml PS compared to transduction with virus only. Analysis was performed by ddPCR on 9/10 post transduction on PBMCs from independent donors. The VCN of cells transduced with transduction enhancer was compared to cells transduced with virus only. This ratio gives the relative transduction enhancement. Bars show mean values from 3 different donors, error bars show standard deviations. P-values are calculated with a 2-tailed student's t-test.

The Examples illustrate the invention:

**Example 1**

Material and Methods

*Cell lines*

Human ovarian carcinoma cells A2780 (Sigma Aldrich) and human lung adenocarcinoma cells NCI H1838 (ATTC) were grown in RPMI 1640 medium supplemented with 10% (vol/vol) fetal calf serum (FCS; Biochrom) and 2 mM L-glutamine (PAN Biotech).

Human cutaneous T lymphocytes HuT78 (ATCC) were grown in IMDB (Invitrogen) medium supplemented with 20% FCS (Biochrom).

Human Chronic B cell leukemia (JVM-3) and human B cell lymphoma (DoHH-2) were grown in RPMI1640 medium supplemented with 10% FCS (Biochrom).

*Lentivirus production*

The lentiviral transduction vectors p-Ubic-Luc2-IRES-Puro and p-CMV-copGFP-IRES-Puro allow expression of Luciferase driven by Ubic promoter or copGFP by CMV promoter, respectively. Replication-defective lentiviral particles were produced by transient co-transduction of HEK293-TN cells in a T75 flask with 10 µg of packaging plasmids pMDL, pVSV-G, pRev (Roche) and 2 µg of transduction vector using Lipofectamine 2000 (Life Technologies) as transduction reagent according to the manufacturer's instructions. The virus particles were harvested 48 hours after transduction, cleared of cell debris by low-speed centrifugation and precipitated with PEG. Viral particles were concentrated by centrifugation and stored at -80°C.

Lentiviral transduction of cell lines

Adherent cells were seeded at 1,5E+05 per well into 96 well plates in appropriate culture medium. After 24-hour incubation (at 37°C with 5% $CO_2$) medium was exchanged and cells were treated with defined adjuvant (i.e. poloxamine) concentrations as indicated. Cells were then transduced with a lentiviral (LV) vector expressing Luciferase or copGFP at MOI 15 (multiplicity of infection - infectious viral particles per cell) and cultured for 72 hours in a cell culture incubator at 37°C and 5% $CO_2$ without change of medium. Suspension cells were seeded as described above and transduced the same days they were seeded with the same LV as described above at MOI15. In given case, plates with seeded cells were centrifuged at 800g for 60 min directly after transduction (spinoculation).

*Quantification of transduction efficiency*

To quantify total viral transgene expression by Luciferase, 100µl Steady-Glo reagent (Promega) was added to the cells 3 days after viral transduction. The plate with the cells was put into a Tecan Infinite M200 plate reader and shaken for 30 seconds. After incubation for 10 minutes at room temperature, luminescence signal was measured by the plate reader. Measurements are presented in relative light units (RLU).

To Analyze transduction efficiency of cells transduced with a copGFP expressing vector, random images per well were taken with a fluorescent microscope with the 10x objective (Leica DM IL LED with a DFC 3000G camera) 3 days after transduction. Cells were counterstained by adding 1µg/ml Hoechst 33342 to the medium and incubation for 15min at 37°C. copGFP and Hoechst expressing cells were counted and transduction efficiency calculated dividing the number of GFP expressing cells by the number of Hoechst stained cells.

*MTT assay (cell viability)*

Cytotoxicity of adjuvants was tested by the colorimetric MTT assay. A2780 cells were seeded into a 96 well plate and cells were treated with adjuvants at concentrations ranging from 0,031mg/ml to 20 ml/ml. After 72 hours incubation wwat 37°C with 5% CO2, 20µl MTT reagent (2,5mg/ml) was added to the cells and cells were incubated for 4 hours at 37°C. 100µl SDS lysis solution (10% SDS in 0,001N HCl) was added per well and incubated for 24 hours at 37°C. Absorbance was measured on a Tecan plate reader at 560/690nm. NAD(P)H-dependent cellular oxidoreductase enzymes in viable, healthy cells reduce MTT to its insoluble form, which can be measured in a plate reader; a reduction in absorbance indicates a reduced number of healthy cells.

*Material and Methods for Example 3*

Human CD34+ hematopoietic stem cells were isolated from peripheral blood mononuclear cells (PBMCs) from three independent donors. For transduction the cells were seeded at 2x10e6 cells per well into 12-well plates in StemMACS HSC Expansion Media XF supplemented with StemMACS HSC Expansion Cocktail. The day after seeding cells were transduced at MO11 with a Lentiviral vector expressing GFP under a CMV promoter. Before transduction, Poloxamine T1107 was added at a final concentration of 1 mg/ml either alone or in combination with 5 µg/ml Protamine sulfate (PS). For each donor a control transduction with virus without enhancer was performed (virus only). For transduction, spinoculation at 600g for 90min at room temperature was applied.

At 24h post transduction, cells were washed twice with PBS and medium was exchanged. On day 9/10 post transduction the percentage of GFP expressing, transduced cells was analyzed by FACS analysis.

Additionally on day 9/10 post transduction, genomic DNA (gDNA) was isolated from the transduced and control cells using QIAmp DNA micro Kit and the number of integrated viral vector genomes (vector copy number, VCN) per cell was analyzed by ddPCR. For detection of the integrated viral vector genome, ddPCR PRE specific primers and as control the single copy gene RPP30 were used (Hauber et al. 2018).

**Example 2**

Results

*Poloxamines enhance transduction in adherent cells*

To analyze the effect of Poloxamines on transduction efficiency of Lentiviral vectors, Poloxamines T908, T1107, T1307 were added to adherent A2780 cells at the indicated concentrations following transduction of these cells with a Lentivirus expressing Luciferase at MO115. As controls virus only (with H2O instead of adjuvant), or 8 mg/ml Polybrene was added to the wells before transduction. All conditions were set up in triplicate. Three days post transduction the efficiency of transduction was assessed by measuring luciferase activity on a plate reader. Results are presented in relative luminescence units (RLU). At concentrations between 5 mg/ml and 0.31 mg/ml transgene expression after transduction was improved up to 2.13 fold, 2.55 fold or 2.91 fold compared to virus only for T908, T1107 and T1307, respectively,. The best improvement for all polymers on A2780 cells can be seen at 0,63mg/ml (Fig. 1a and 1b).

This transduction test was also replicated in the adherent human lung adenocarcinoma cell line NCI-H1838. In general, transduction level measured by luciferase activity was lower than in A2780 cells (Fig. 1 c). We could still observe that the addition of Poloxamines increased transduction efficiency up to 2.86-fold (T1107 at 0.63 mg/ml), 2.77-fold (T1307 at 0.31 mg/ml) and 2.68-fold (T908 at 0.31 mg/ml) compared to transduction with virus without transduction enhancer (Fig.

1 d).

*Poloxamines and protamine sulfate have an additive effect on transduction enhancement*

To measure additive effects, A2780 cells were transduced with copGFP expressing Lentivirus with the addition of Poloxamines T1107, T1307 or T908 at 1 mg/ml. Additionally, a combination of 1 mg/ml of each Poloxamine with 5 µg/ml or 40 µg/ml Protamine sulfate or 1 mg/ml of Poloxamine were used to analyze synergistic effects. In each well, 6 images were taken (examples of T1107 shown in Fig 2d), copGFP expressing cells were counted and compared to total cells stained with Hoechst 33342 dye. Mean percentages of GFP expressing cells and standard deviations from 3 wells per condition are shown in Fig. 2a-c. All transduction enhancers used alone increased lentiviral transduction compared to virus only by 1.67-fold (Polybrene) (Fig. 2a-c). Poloxamines T1107, T1307 and T908 at 1 mg/ml each increased the percentage of transduced cells by 2.46-fold, 2.54-fold and 2.68-fold respectively compared to virus only. Interestingly we found that with T1107 in combination with 40 µg/ml Protamine sulfate 1.43-fold more cells are transduced than with T1107 alone (Fig. 2a). Also for T1307 and T908 we observed a significantly improved transduction rate with Poloxamine in combination with 40 µg/ml Protamine sulfate over Poloxamine alone by 1.49-fold and 1.71-fold respectively (Fig. 2b and c). This improvement can also be seen compared to 40 µg/ml Protamine sulfate alone. The combinations of Poloxamines with other Poloxamines did not give any significant improvement.

To confirm this additive effect also on a total transgene expression level, lentiviral transduction was performed with a lentivirus expressing Luciferase. Concentrations and combinations of Poloxamines and all other transduction enhancers were used as described in the experiment above.

Transduction enhancers alone increased total transgene expression 1.14-fold (5µg/ml Protamine sulfate) to 2.08-fold (40 µg/ml Protamine sulfate) (Fig. 2 e-g). Poloxamines T1107, T1307 and T908 increased the transgene expression level by 1.54, 1.51 and 1.58-fold respectively compared to virus only (Fig. 2 e-g). We were able to confirm an improved transduction of Poloxamines in combination with 40 µg/ml Protamine sulfate compared to Poloxamine alone. T1107, T1307 and T908 in combination with 40µg/ml Protamine sulfate increased transgene expression by 1.96, 2.11 and 1.89-fold respectively compared to Poloxamine alone (Fig 2 e-g). The combinations with other Poloxamines did not give any significant improvement.

*Poloxamines enhance transduction in suspension cells*

Many established tumor cell lines in pre-clinical research offer only poor transduction rates with common viral and non-viral tools. Anaplastic large cell lymphoma (ALCL) cell lines that grow in suspension cultures belong to this hard-to-infect subset of tumor cell lines. With a modified protocol including an optional spinoculation step during LV infection, we tested poloxamines for facilitating lentiviral infection. Enhancement of transduction efficiency by poloxamines with and without spinoculation was first evaluated on HuT78 human cutaneous T lymphocyte suspension cells. Transduction was performed with a lentivirus expressing Luciferase to measure the effect on total transgene expression. Maximal transduction efficiency was reached between 5 mg/ml and 1.25 mg/ml Poloxamine and decreased at lower concentrations. Maximal improvement of Luciferase signal over virus only control was 3.53-fold (T908, 5mg/ml), 5.98-fold (T1107, 2.5 mg/ml) and 4.85-fold (T1307, 2.5 mg/ml) without spinoculation respectively (Fig 3a+c). With spinoculation maximal improvement was 4,08-fold (T908, 2.5 mg/ml), 5.06-fold (T1107 (1.25 mg/ml) and 4.32-fold (T1307, 1.25 mg/ml) respectively (Fig 3b+d). In general, expression levels of the transgene were substantially lower compared to adherent A2780 cells. With spinoculation expression levels were increased up to 2.6-fold compared to without spinoculation (Fig 3c compared to 3d) but total transgene expression levels were still relatively low compared to adherent cells (Fig 1).

We also tested two additional suspension cell lines, JVM-3 (human chronic B cell leukemia) and DoHH-2 (human B cell lymphoma). In JVM-3 cells we observed a maximal enhancement of transduction of 2.46-fold with T908 at 20 mg/ml, 2.17-fold with T1107 at 10 mg/ml and 2.47-fold with T1307 at 20 mg/ml (Fig 4 a+b). In DoHH-2 cells a 2.26-fold (T908 at 20 mg/ml), 2.42-fold (T1107 at 20 mg/ml) and 3.29-fold (T1307 at 20 mg/ml) enhancement compared to virus only was observed respectively (Fig 5a+b). JVM-3 cells had a higher total transgene expression level compared to DoHH-2 in all conditions used. For both, JVM-3 and DoHH-2, suspension cell lines the strongest effect was seen at concentrations between 10mg/ml and 20mg/ml. In general, these two suspension cell lines also had significantly lower transgene expression levels after transduction compared to adherent A2780 cells.

*Cell viability*

For the application of transduction enhancers in gene therapeutic applications, it is important that the substances used do not affect viability and do not have a toxic effect on the cells.

MTT assay is a colorimetric assay to assess metabolic activity in cells. The tetrazolium dye MTT (3-(4,5-dimethylthiazol-2-yl)-2,5-diphenyltetrazolium bromide) is reduced by cellular oxidoreductase enzymes to it insoluble form formazan, which has a purple color and can be quantified by absorbance measurement. To test the effect on the viability of cells, Poloxamines at final concentrations used for transduction enhancement, between 20 mg/ml and 0,31mg/ml, were added to the cells and viability was measured after 3 days by MTT assay. Absorbance values were then compared to Medium only or water control. For T908 viability was between 69 and 90% with the highest viability observed at 0.31 mg/ml. Morphologically we did not observe apoptosis in the cells. For T1107 viability was between 99% and 116% with the highest viability at 20 mg/ml. Similar results can be seen for T1307 with a viability between 84% (0.31 mg/ml) and 109% (20 mg/ml) (Fig. 3(a) and (b)). Values above 100% indicate that these cells have more enzymatic activity and higher viability than the control. For T1107 and T1307, viability values over 100% at high concentrations over 5mg/ml could indicate, that these Poloxamines have a positive effect on the growth or enzymatic activity of the cells. The lower viability for T908 does not clearly point towards a toxic effect since we could not observe morphological signs of apoptosis. This could rather indicated that the metabolic activity of the cells was affected by T908.

Example 3

*Enhancement of transduction and VCN*

To measure improvement of transduction by Poloxamine T1107, human CD34+ PBMCs from 3 different donors were harvested and seeded into 12- well plates at 2x10e6 cells per well. On the next day, cells were transduced with a Lentivirus expressing GFP at MOI1 using spinoculation at 600g for 90 min. As transduction enhancer 1 mg/ml T1107 either alone or in combination with 5 $\mu$g/ml Protamine sulfate (PS) was added before the virus. 9/10 days after transduction the percentage of GFP expressing (GFP+) cells was measured by FACS analysis. The percentage of transduced GFP+ cells with transduction enhancer was compared to GFP+ cells transduced with virus only. This ratio gives the relative transduction enhancement.

With 1 mg/ml T1107 alone a significant transduction enhancement of 3.95-fold compared to virus only on day 9/10 was observed. T1107 in combination with 5 $\mu$g/ml PS further improved transduction 5.09-fold compared to virus only (Fig. 1). This result shows the effect of T1107 in combination with PS with a 28.6% better transduction improvement over T1107 alone, respectively.

Additionally, the vector copy number (VCN) per cell was analyzed on day 9/10 after transduction by ddPCR. The VCN number describes the average number of transgene copies, which is integrated into a target cell. By transduction with virus alone a VCN of 0.07 was reached, whereas addition of 1 mg/ml T1107 resulted in a VCN of 0.3. Combination of T1107 with 5 $\mu$g/ml PS further increased the VCN to 0.43. Adding T1107 either alone or in combination with PS increased the VCN 4.44-fold and 6.28-fold respectively compared to virus only (Fig 2). The combination of T1107 with PS resulted in a 41.2% better VCN improvement than use of T1107 alone. These results clearly demonstrate that addition of 1 mg/ml T1107 significantly enhances the VCN in lentiviral transduction over transduction with virus alone. Furthermore, the combination of T1107 with PS results in an additional increase in VCN.

**References**

Alakhov, Valery, Evgueni Klinski, Shengmin Li, and Grzegorz Pietrzynski. 1999. "Block Copolymer-Based Formulation of Doxorubicin. From Cell Screen to Clinical Trials." Colloids and Surfaces B: Biointerfaces 16: 113-34.

Alvarez-Lorenzo, Carmen et al. 2010. "Poloxamine-Based Nanomaterials for Drug Delivery." Frontiers in biosciences 2: 424-40.

Anastasov, Natasa et al. 2009. "Efficient ShRNA Delivery into B and T Lymphoma Cells Using Lentiviral Vector-Mediated Transfer." Journal of hematopathology 2(1): 9-19. 2010. "C/EBPbeta Expression in ALK-Positive Anaplastic Large Cell Lymphomas Is Required for Cell Proliferation and Is Induced by the STAT3 Signaling Pathway." Haematologica 95(5): 760-67.

Aubin, R J, M Weinfeld, and M C Paterson. 1988. "Factors Influencing Efficiency and Reproducibility of Polybrene-Assisted Gene Transfer." Somatic cell and molecular genetics 14(2): 155-67.

Bartel, David P. 2009. "MicroRNAs: Target Recognition and Regulatory Functions." Cell 136(2): 215-33.

Bischof, Daniela, and Kenneth Cornetta. 2010. "Flexibility in Cell Targeting by Pseudotyping Lentiviral Vectors." Methods in molecular biology (Clifton, N.J.) 614: 53-68.

Bukrinsky, M I et al. 1993. "A Nuclear Localization Signal within HIV-1 Matrix Protein That Governs Infection of Non-Dividing Cells." Nature 365(6447): 666-69.

Burns, J C et al. 1993. "Vesicular Stomatitis Virus G Glycoprotein Pseudotyped Retroviral Vectors: Concentration to Very High Titer and Efficient Gene Transfer into Mammalian and Nonmammalian Cells." Proceedings of the National Academy of Sciences of the United States of America 90(17): 8033-37.

Castro, B A, C D Weiss, L D Wiviott, and J A Levy. 1988. "Optimal Conditions for Recovery of the Human Immunodeficiency Virus from Peripheral Blood Mononuclear Cells." Journal of clinical microbiology 26(11): 2371-76.

Chiappetta, Diego A., and Alejandro Sosnik. 2007. "Poly(Ethylene Oxide)-Poly(Propylene Oxide) Block Copolymer Micelles as Drug Delivery Agents: Improved Hydrosolubility, Stability and Bioavailability of Drugs." European Journal of Pharmaceutics and Biopharmaceutics 66(3): 303-17.

Cho, Eunhee, Jeoung Soo Lee, and Ken Webb. 2012. "Formulation and Characterization of Poloxamine-Based Hydrogels as Tissue Sealants." Acta Biomaterials 8(6): 2223-32.

Chu, Kang, Kenneth G Cornetta, and Michael J Econs. 2008. "Efficient and Stable Gene Expression into Human Osteoclasts Using an HIV-1-Based Lentiviral Vector." DNA and cell biology 27(6): 315-20.

Dull, T et al. 1998. "A Third-Generation Lentivirus Vector with a Conditional Packaging System." Journal of virology 72(11): 8463-71.

Erukova, Veronika Yu, Oksana O. Krylova, Yuri N. Antonenko, and Nickolay S. Melik-Nubarov. 2000. "Effect of Ethylene Oxide and Propylene Oxide Block Copolymers on the Permeability of Bilayer Lipid Membranes to Small Solutes Including Doxorubicin." Biochimica et Biophysica Acta - Biomembranes 1468(1-2): 73-86.

Fenard, David et al. 2013. "Vectofusin-1, a New Viral Entry Enhancer, Strongly Promotes Lentiviral Transduction of Human Hematopoietic Stem Cells." Molecular Therapy - Nucleic Acids 2(March): e90.

Funke, Sabrina et al. 2008. "Targeted Cell Entry of Lentiviral Vectors." Molecular therapy: the journal of the American Society of Gene Therapy 16(8): 1427-36.

Gruber, A et al. 2000. "Dendritic Cells Transduced by Multiply Deleted HIV-1 Vectors Exhibit Normal Phenotypes and Functions and Elicit an HIV-Specific Cytotoxic T-Lymphocyte Response in Vitro." Blood 96(4): 1327-33.

Gundry, Michael C, Lorenzo Brunetti, Angelique Lin, and Allison E Mayle. 2016. "Highly Efficient Genome Editing of Murine and Human Hematopoietic Progenitor Cells by CRISPR/Cas9." Cell Reports 17(5): 1453-61.

Guo, J., W. Wang, D. Yu, and Y. Wu. 2011. "Spinoculation Triggers Dynamic Actin and Cofilin Activity That Facilitates HIV-1 Infection of Transformed and Resting CD4 T Cells." Journal of Virology 85(19): 9824-33. http://jvi.asm.org/cgi/doi/10.1128/JVI.05170-11.

Hansen, Scott G et al. 2011. "Profound Early Control of Highly Pathogenic SIV by an Effector Memory T-Cell Vaccine." Nature 473(7348): 523-27.

Harada, Atsushi, and Kazunori Kataoka. 1995. "Formation of Polyion Complex Micelles in an Aqueous Milieu from a Pair of Oppositely-Charged Block Copolymers with Poly(Ethylene Glycol) Segments." Macromolecules 28(15): 5294-99. https://doi.org/10.1021/ma00119a019.

Hargus, Gunnar et al. 2010. "Differentiated Parkinson Patient-Derived Induced Pluripotent Stem Cells Grow in the Adult Rodent Brain and Reduce Motor Asymmetry in Parkinsonian Rats." Proceedings of the National Academy of Sciences of the United States of America 107(36): 15921-26.

Hauber, Ilona et al. 2018. "Improving Lentiviral Transduction of CD34+ Hematopoietic Stem and Progenitor Cells." Human Gene Therapy Methods: hgtb.2017.085. http://www.liebertpub.com/doi/10.1089/hgtb.2017.085.

Heffner, Garrett C. et al. 2018. "Prostaglandin E2 Increases Lentiviral Vector Transduction Efficiency of Adult Human Hematopoietic Stem and Progenitor Cells." Molecular Therapy 26(1): 320-28. https://doi.org/10.1016/j.ymthe.2017.09.025.

Hesse, J, P Ebbesen, and G Kristensen. 1978. "Correlation between Polyion Effect on Cell Susceptibility to in Vitro Infection with Murine C-Type Viruses and Polyion Effect on Some Membrane-Related Functions." Intervirology 9(3): 173-83.

Ingrao, Dina et al. 2014. "Concurrent Measures of Fusion and Transduction Efficiency of Primary CD34+ Cells with Human Immunodeficiency Virus 1-Based Lentiviral Vectors Reveal Different Effects of Transduction Enhancers." Human Gene Therapy Methods 25(1): 48-56. http://online.liebertpub.com/doi/10.1089/hgtb.2013.090.

Jacome, Ariana et al. 2009. "Lentiviral-Mediated Genetic Correction of Hematopoietic and Mesenchymal Progenitor Cells from Fanconi Anemia Patients." Molecular therapy: the journal of the American Society of Gene Therapy 17(6): 1083-92.

Jaenisch, Rudolf, and Richard Young. 2008. "Stem Cells, the Molecular Circuitry of Pluripotency and Nuclear Reprogramming." Cell 132(4): 567-82.

Kabanov, Alexander, Jian Zhu, and Valery Alakhov. 2005. "Pluronic Block Copolymers for Gene Delivery." Advances in genetics 53(05).

Khan, Faheem Ahmed et al. 2016. "CRISPR / Cas9 Therapeutics: A Cure for Cancer and Other Genetic Diseases." Oncotarget 7(32): 52541-52.

Kowal, Joanna, Mercedes Tkach, and Clotilde Thery. 2014. "Biogenesis and Secretion of Exosomes." Current Opinion in Cell Biology 29(1): 116-25.

Lee, Hyun Joo et al. 2009. "Retronectin Enhances Lentivirus-Mediated Gene Delivery into Hematopoietic Progenitor Cells." Biologicals 37(4): 203-9. http://dx.doi.org/10.1016/j.biologicals.2009.01.008.

Lemieux, P, and N Guerin. 2000. "A Combination of Poloxamers Increases Gene Expression of Plasmid DNA in Skeletal Muscle." Gene Therapy 7: 986-91.

Lewis, Gretchen et al. 2018. "Staurosporine Increases Lentiviral Vector Transduction Efficiency of Human Hematopoietic Stem and Progenitor Cells." Molecular Therapy - Methods and Clinical Development 9(June): 313-22. https://doi.org/10.1016/j.omtm.2018.04.001.

Lin, Huan-Ting et al. 2016. "Application of Droplet Digital PCR for Estimating Vector Copy Number States in Stem Cell Gene Therapy." Human gene therapy methods 27(5): 197-208.

Mahajan, Vivek et al. 2017. "Horizontal Gene Transfer from Macrophages to Ischemic Muscles upon Delivery of Naked DNA with Pluronic Block Copolymers." Blomaterials 1(919): 58-70.

Marconi, Peggy, Roberto Manservigi, and Alberto L Epstein. 2010. "HSV-1-Derived Helper-Independent Defective Vectors, Replicating Vectors and Amplicon Vectors, for the Treatment of Brain Diseases." Current opinion in drug discovery & development 13(2): 169-83.

Matrai, Janka, Marinee K L Chuah, and Thierry VandenDriessche. 2010. "Recent Advances in Lentiviral Vector Development and Applications." Molecular therapy: the journal of the American Society of Gene Therapy 18(3): 477-90.

Meyer, Conary et al. 2017. "Pseudotyping Exosomes for Enhanced Protein Delivery in Mammalian Cells." International Journal of Nanomedicine 12.

Millington, Michelle et al. 2009. "Towards a Clinically Relevant Lentiviral Transduction Protocol for Primary Human CD34 Hematopoietic Stem/Progenitor Cells." P/oS one 4(7): e6461.

Mohr, Christian Andreas et al. 2010. "A Spread-Deficient Cytomegalovirus for Assessment of First-Target Cells in Vaccination." Journal of virology 84(15): 7730-42.

Morrell, Nathan T. et al. 2008. "Liposomal Packaging Generates Wnt Protein with in Vivo Biological Activity." PLoS ONE 3(8).

Moulay, Gilles et al. 2017. "Histidine-Rich Designer Peptides of the LAH4 Family Promote Cell Delivery of a Multitude of Cargo." Journal of Peptide Science 23(4): 320-28.

Murray, Lesley et al. 1999. "Optimization of Retroviral Gene Transduction of Mobilized Primitive Hematopoietic Progenitors by Using Thrombopoietin, FIt3, and Kit Ligands and RetroNectin Culture." Human Gene Therapy 10: 1743-52.

O'Connell, Ryan M et al. 2010. "Lentiviral Vector Delivery of Human Interleukin-7 (HIL-7) to Human Immune System (HIS) Mice Expands T Lymphocyte Populations." PloS one 5(8): e12009.

O'Loughlin, Aisling, Caroline Woffindale, and Matthew Wood. 2012. "Exosomes and the Emerging Field of Exosome-Based Gene Therapy." Current Gene Therapy 12(4): 262-74. http://www.eurekaselect.com/openurl/content.php?genre=article&issn=1566-5232&volume=12&issue=4&spage=262.

Poczobutt, Joanna M et al. 2010. "Benign Mammary Epithelial Cells Enhance the Transformed Phenotype of Human Breast Cancer Cells." BMC cancer 10: 373.

Ricciotti, Emanuele and FitzGerald, Garret A. 2011. "Prostaglandins and Inflammation." Arterioscler Thromb Vasc Boil 31(5): 986-1000. https://doi.org/10.1161/ATVBAHA.110.207449.

Robert, Marc-Andre et al. 2017. "Virus-Like Particles Derived from HIV-1 for Delivery of Nuclear Proteins: Improvement of Production and Activity by Protein Engineering." Molecular biotechnology 59(1): 9-23.

Rouas, Redouane et al. 2002. "Lentiviral-Mediated Gene Delivery in Human Monocyte-Derived Dendritic Cells: Optimized Design and Procedures for Highly Efficient Transduction Compatible with Clinical Constraints." Cancer gene therapy 9(9): 715-24.

Schmolka, Irving R. 1977. "A Review of Block Polymer Surfactants." Journal of the American Oil Chemists' Society 54(3): 110-16.

Schwarzbauer, Jean E., and Douglas W. DeSimone. 2011. "Fibronectins, Their Fibrillogenesis, and in Vivo Functions." Cold Spring Harbor Perspectives in Biology 3(7): 1-19.

Serra-Gómez, Rafael, Cecile A. Dreiss, Javier Gonzalez-Benito, and Gustavo Gonzalez-Gaitano. 2016. "Structure and Rheology of Poloxamine T1107 and Its Nanocomposite Hydrogels with Cyclodextrin-Modified Barium Titanate Nanoparticles." Langmuir 32(25): 6398-6408.

Song, Hongmei et al. 2014. "Pluronic L64-Mediated Stable HIF-1 a Expression in Muscle for Therapeutic Angiogenesis in Mouse Hindlimb Ischemia." International journal of nanomedicine 9: 3439-52.

Subbaraman, Lakshman N et al. 2006. "Rewetting Drops Containing Surface Active Agents Improve the Clinical Performance of Silicone Hydrogel Contact Lenses." Optometry and vision science: official publication of the American Academy of Optometry 83(3): 143-51.

Takahashi, Kazutoshi, and Shinya Yamanaka. 2006. "Induction of Pluripotent Stem Cells from Mouse Embryonic and Adult Fibroblast Cultures by Defined Factors." Cell 126(4): 663-76.

Tamaoki, T, and H Nakano. 1990. "Potent and Specific Inhibitors of Protein Kinase C of Microbial Origin." Bio/technology (Nature Publishing Company) 8(8): 732-35.

Tonge, S, L Jones, S Goodall, and B Tighe. 2001. "The Ex Vivo Wettability of Soft Contact Lenses." Current eye research 23(1): 51-59.

Tilysuz, Nesrin et al. 2017. "Lipid-Mediated Wnt Protein Stabilization Enables Serum-Free Culture of Human Organ Stem Cells." Nature Communications 8: 14578. http://www.ncbi.nlm.nih.gov/pubmed/28262686%0Ahttp://www.nature.com/doifinder/10 .1038/ncomms14578.

Wingerath, Jessica et al. 2017. "Recombinant LCMV Vectors Induce Protective Immunity Following Homologous and Heterologous Vaccinations." Molecular therapy: the journal of the American Society of Gene Therapy 25(11): 2533-45.

Wurm, Melanie et al. 2010. "The Influence of Semen-Derived Enhancer of Virus Infection on the Efficiency of Retroviral Gene Transfer." The journal of gene medicine 12(2): 137-46.

Zhang, Bingqi, Mathumai Kanapathipillai, Paul Bisso, and Surya Mallapragada. 2009. "Novel Pentablock Copolymers for Selective Gene Delivery to Cancer Cells." Pharmaceutical Research 26(3): 700-713.

**Claims**

1. A composition comprising or consisting of

    (a) a poloxamine;
    (b)

        (i) a retroviral vector; and
        (ii) a vertebrate cell.

2. The composition of claim 1, wherein

    (a) said poloxamine is a sequential poloxamine; and/or
    (b) has a structure of formula (I)

$$R^1R^2N\text{-}(CH_2)_2\text{-}NR^3R^4 \qquad (I)$$

    or formula (II)

$$R^1R^2R^5N^+\text{-}(CH_2)_2\text{-}N^+R^3R^4R^6 \ C\text{-}I \qquad (II)$$

    wherein in either formula
    $R^1$ is $H\text{-}(O(CH_2)_2)_a\text{-}(OCH(CH_3)CH_2)_b\text{-}$;
    $R^2$ is $H\text{-}(O(CH_2)_2)_c\text{-}(OCH(CH_3)CH_2)_d\text{-}$;
    $R^3$ is $\text{-}(CH_2CH(CH_3)O)_e\text{-}((CH_2)_2O)_f\text{-}H$;
    $R^4$ is $\text{-}(CH_2CH(CH_3)O)_g\text{-}((CH_2)_2O)_h\text{-}H$;
    $R^5$ and $R^6$ are independently absent or selected from H, $C_1$ to $C_6$ alkyl, $C_2$ to $C_6$ alkenyl and $C_2$ to $C_6$ alkinyl;
    wherein if $R^5$ or $R^6$ are absent, the N bound to $R^5$ or $R^6$ does not carry a positive charge;
    C-I stands for one or more counter ions which render the structure of formula (II) electroneutral;
    a, c, f and h are independently an integer from about 25 to about 200, preferably from about 50 to about 120; and
    b, d, e and g are independently an integer from about 5 to about 50, preferably from about 10 to about 25, wherein "about" refers to +/- 30%.

3. The composition of claim 2(b), wherein

    a = c; f = h; b = d; and/or e = g; and preferably
    a = c = f = h; and/or b = d = e = g.

4. The composition of claim 2(b) or 3, wherein the propylene oxide - ethylene oxide ratio (#PO/#EO) defined as

$$(b + d + e + g) / (a + c + f + h)$$

    is in the range from about 0.05 to about 1.5, preferably from about 0.1 to about 1.0, more preferably from about 0.15 to about 0.67, and most preferably from about 0.18 to about 0.35, wherein "about" refers to +/- 30%.

5. The composition of any one of claims 1 to 4, wherein said composition furthermore comprises or furthermore consists of

  (a) one or more polycationic substances selected from the group of polycationic polymers or polycationic peptides;
  (b) prostaglandins;
  (c) glycoproteins;
  (d) poloxamers; and/or
  (e) cyclosporins.

6. A pharmaceutical composition comprising or consisting of the composition of any one of claims 1 to 5.

7. The pharmaceutical composition of claim 6, wherein said retroviral vector carries a gene which is beneficial for an individual suffering from a disease or being at risk of developing said disease.

8. In vitro or ex vivo use of poloxamine for transducing a vertebrate cell with a retroviral vector, wherein optionally furthermore use is made of

  (a) one or more polycationic substances selected from the group of polycationic polymers or polycationic peptides;
  (b) prostaglandins;
  (c) glycoproteins;
  (d) poloxamers; and/or
  (e) cyclosporins,

wherein said use does not involve a process for modifying the germ line genetic identity of human beings.

9. An in vitro or ex vivo method of transducing a vertebrate cell with a retroviral vector, said method comprising or consisting of bringing into contact said vertebrate cell, a poloxamine, and said viral vector,

  wherein preferably said bringing into contact is effected in the following order:

    (a) providing said cell;
    (b) adding said poloxamine; and
    (c) adding said vector,

  wherein preferably after said bringing into contact spinoculation is performed,
  wherein said method is not a process for modifying the germ line genetic identity of human beings.

10. The method of claim 9, wherein transduction is enhanced as compared to a reference method, which reference method is without adding said poloxamine, said reference method being otherwise identical to said method of transducing a vertebrate cell.

11. A method of enhancing viral copy number (VCN), said method comprising the method of claim 9, wherein VCN is enhanced as compared to a reference method, which reference method is without adding said poloxamine, said reference method being otherwise identical to said method of enhancing VCN, wherein preferably the enhancement of VCN is at least 1.5-fold, at least 2-fold, at least 3-fold, at least 5-fold or at least 10-fold.

12. The method of any one of claims 9 to 11, wherein

  (a) said retroviral vector is a lentiviral vector; and/or
  (b) said poloxamine is T1107.

13. The method of any one of claims 9 to 12, wherein said bringing into contact comprises or further consists of bringing into contact with

  (a) one or more polycationic substances selected from the group of polycationic polymers or polycationic peptides;

(b) prostaglandins;
(c) glycoproteins;
(d) poloxamers; and/or
(e) cyclosporins.

14. The composition of any one of claims 1 to 7, wherein said retroviral vector is a lentiviral vector;

said poloxamine is provided in a concentration in the range from about 0.1 to about 40 mg/ml, preferably from about 0.2 to about 30 mg/ml, more preferably between about 0.3 and about 20 mg/ml, wherein

(a) if said cell is a cell in suspension, about 5 to about 20 mg/ml is preferred; and
(b) if said cell is adherent, about 0.3 to about 10 mg/ml is preferred; and/or
(a) said polycationic polymers are selected from 1,5-dimethyl-1,5-diaza-undeca-methyl-polymethobromide (Polybrene) and poly(ethylene glycol)-poly(L-lysine) block copolymer (PEG-PLL);
(b) said polycationic peptides are selected from protamine sulfate, poly-L-lysin (PLL) having a mean molecular weight from about 1 to about 300 kDa and Vectofusin-1®;
(c) said glycoprotein is fibronectin (RetroNectin®);
(d) said prostaglandine is PGE2;
(e) said poloxamer is Synperonic® F108 or Synperonic® F98; and/or
(f) said cyclosporine is CsA or CsH,

wherein "about" refers to +/- 30%.

15. The use of claim 8, wherein

said retroviral vector is a lentiviral vector;
said poloxamine is provided in a concentration in the range from about 0.1 to about 40 mg/ml, preferably from about 0.2 to about 30 mg/ml, more preferably between about 0.3 and about 20 mg/ml, wherein

(a) if said cell is a cell in suspension, about 5 to about 20 mg/ml is preferred; and
(b) if said cell is adherent, about 0.3 to about 10 mg/ml is preferred.
(a) said polycationic polymers are selected from 1,5-dimethyl-1,5-diaza-undeca-methyl-polymethobromide (Polybrene) and poly(ethylene glycol)-poly(L-lysine) block copolymer (PEG-PLL);
(b) said polycationic peptides are selected from protamine sulfate, poly-L-lysin (PLL) having a mean molecular weight from about 1 to about 300 kDa and Vectofusin-1®;
(c) said glycoprotein is fibronectin (RetroNectin®);
(d) said prostaglandine is PGE2;
(e) said poloxamer is Synperonic® F108 or Synperonic® F98; and/or
(f) said cyclosporine is CsA or CsH

wherein "about" refers to +/- 30%.

16. The method of any one of claims 9 to 13, wherein

said retroviral vector is a lentiviral vector;
said poloxamine is provided in a concentration in the range from about 0.1 to about 40 mg/ml, preferably from about 0.2 to about 30 mg/ml, more preferably between about 0.3 and about 20 mg/ml, wherein

(a) if said cell is a cell in suspension, about 5 to about 20 mg/ml is preferred; and
(b) if said cell is adherent, about 0.3 to about 10 mg/ml is preferred; and/or

(a) said polycationic polymers are selected from 1,5-dimethyl-1,5-diaza-undeca-methyl-polymethobromide (Polybrene) and poly(ethylene glycol)-poly(L-lysine) block copolymer (PEG-PLL);
(b) said polycationic peptides are selected from protamine sulfate, poly-L-lysin (PLL) having a mean molecular weight from about 1 to about 300 kDa and Vectofusin-1®;
(c) said glycoprotein is fibronectin (RetroNectin®);
(d) said prostaglandine is PGE2;
(e) said poloxamer is Synperonic® F108 or Synperonic® F98; and/or

(f) said cyclosporine is CsA or CsH,

wherein "about" refers to +/- 30%.

**Patentansprüche**

1. Zusammensetzung, umfassend oder bestehend aus

    (a) einem Poloxamin;
    b)

        (i) einem retroviralen Vektor; und
        (ii) einer Wirbeltierzelle.

2. Zusammensetzung nach Anspruch 1, wobei

    a) das Poloxamin ein sequentielles Poloxamin ist; und/oder
    b) eine Struktur der Formel (I)

$$R^1R^2N\text{-}(CH_2)_2\text{-}NR^3R^4 \qquad (I)$$

    oder Formel (II) aufweist

$$R^1R^2R^5N^+\text{-}(CH_2)_2\text{-}N^+R^3R^4R^6 \ C\text{-}I \qquad (II)$$

    wobei in beiden Formeln

    $R^1$ is $H\text{-}(O(CH_2)_2)_a\text{-}(OCH(CH_3)CH_2)_b\text{-}$;
    $R^2$ is $H\text{-}(O(CH_2)_2)_c\text{-}(OCH(CH_3)CH_2)_d\text{-}$;
    $R^3$ is $\text{-}(CH_2CH(CH_3)O)_e\text{-}((CH_2)_2O)_r\text{-}H$;
    $R^4$ is $\text{-}(CH_2CH(CH_3)O)_g\text{-}((CH_2)_2O)_h\text{-}H$;
    $R^5$ und $R^6$ unabhängig voneinander entweder nicht vorhanden oder ausgewählt sind aus H, $C_1$ bis $C_6$ Alkyl, $C_2$ bis $C_6$ Alkenyl und $C_2$ bis $C_6$ Alkinyl; wobei, falls $R^5$ oder $R^6$ nicht vorhanden sind, das an $R^5$ oder $R^6$ gebundene N keine positive Ladung trägt;
    Cl für ein oder mehrere Gegenionen steht, welche die Struktur der Formel (II) elektroneutral machen;
    a, c, f und h sind unabhängig voneinander eine ganze Zahl von etwa 25 bis etwa 200, vorzugsweise von etwa 50 bis etwa 120 sind; und
    b, d, e und g sind unabhängig voneinander eine ganze Zahl von etwa 5 bis etwa 50, vorzugsweise von etwa 10 bis etwa 25 sind, wobei sich "etwa" auf +/- 30% bezieht.

3. Zusammensetzung nach Anspruch 2(b), wobei

    a = c; f = h; b = d; und/oder e = g; und vorzugsweise
    a = c = f = h; und/oder b = d = e = g.

4. Zusammensetzung nach Anspruch 2(b) oder 3, wobei das Verhältnis von Propylenoxid zu Ethylenoxid (#PO/#EO) definiert ist als

$$(b + d + e + g) / (a + c + f + h)$$

    im Bereich von etwa 0,05 bis etwa 1,5, vorzugsweise von etwa 0,1 bis etwa 1,0, bevorzugter von etwa 0,15 bis etwa 0,67 und besonders bevorzugt von etwa 0,18 bis etwa 0,35 liegt, wobei sich "etwa" auf +/- 30% bezieht.

5. Zusammensetzung nach einem der Ansprüche 1 bis 4, wobei diese Zusammensetzung darüber hinaus umfasst oder darüber hinaus besteht aus

(a) einer oder mehreren polykationischen Substanzen, ausgewählt aus der Gruppe von polykationischen Polymeren oder polykationischen Peptiden;
b) Prostaglandinen;
(c) Glykoproteinen;
(d) Poloxameren; und/oder
(e) Cyclosporinen.

6. Pharmazeutische Zusammensetzung, welche die Zusammensetzung nach einem der Ansprüche 1 bis 5 umfasst oder daraus besteht.

7. Pharmazeutische Zusammensetzung nach Anspruch 6, wobei der retrovirale Vektor ein Gen trägt, das für eine Person, die an einer Krankheit leidet oder ein Risiko für die Entwicklung dieser Krankheit aufweist, von Vorteil ist.

8. In-vitro- oder Ex-vivo-Verwendung von Poloxamin zur Transduktion einer Wirbeltierzelle mit einem retroviralen Vektor, wobei optional darüber hinaus Gebrauch gemacht wird von

(a) einer oder mehreren polykationischen Substanzen, ausgewählt aus der Gruppe von polykationischen Polymeren oder polykationischen Peptiden;
b) Prostaglandinen;
(c) Glykoproteinen;
(d) Poloxameren; und/oder
(e) Cyclosporinen,

wobei dieser Gebrauch keinen Prozess zur Veränderung der Keimbahn-Genidentität von Menschen einbezieht.

9. In-vitro- oder Ex-vivo-Verfahren zur Transduktion einer Wirbeltierzelle mit einem retroviralen Vektor, wobei das Verfahren das In-Kontakt-Bringen der Wirbeltierzelle, eines Poloxamins und des viralen Vektors umfasst oder daraus besteht,

wobei das In-Kontakt-Bringen vorzugsweise in folgender Reihenfolge erfolgt:

(a) Bereitstellen der Zelle;
b) Hinzufügen des Poloxamins; und
(c) Hinzufügen des Vektors,

wobei vorzugsweise nach In-Kontakt-Bringen die Spinokulation durchgeführt wird,
wobei das Verfahren kein Prozess zur Veränderung der Keimbahn-Genidentität von Menschen ist.

10. Verfahren nach Anspruch 9, wobei Transduktion im Vergleich zu einem Referenzverfahren verbessert ist, wobei das Referenzverfahren ohne Hinzufügen des Poloxamins erfolgt und das Referenzverfahren ansonsten mit dem Verfahren zur Transduktion einer Wirbeltierzelle identisch ist.

11. Verfahren zur Erhöhung der Virusvervielfältigungszahl (VCN), wobei das Verfahren das Verfahren nach Anspruch 9 umfasst, wobei die VCN im Vergleich zu einem Referenzverfahren erhöht wird, wobei das Referenzverfahren ohne Hinzufügen des Poloxamins erfolgt, wobei das Referenzverfahren ansonsten mit dem Verfahren zur Erhöhung der VCN identisch ist, wobei die Erhöhung der VCN vorzugsweise mindestens das 1,5-fache, mindestens das 2-fache, mindestens das 3-fache, mindestens das 5-fache oder mindestens das 10-fache beträgt.

12. Verfahren nach einem der Ansprüche 9 bis 11, wobei

(a) der retrovirale Vektor ein lentiviraler Vektor ist; und/oder
(b) das Poloxamin T1107 ist.

13. Verfahren nach einem der Ansprüche 9 bis 12, wobei das In-Kontakt-Bringen das In-Kontakt-Bringen umfasst oder weiter daraus besteht, mit

(a) einer oder mehreren polykationischen Substanzen, ausgewählt aus der Gruppe von polykationischen Polymeren oder polykationischen Peptiden;

b) Prostaglandinen;
(c) Glykoproteinen;
(d) Poloxameren; und/oder
(e) Cyclosporinen.

**14.** Zusammensetzung nach einem der Ansprüche 1 bis 7, wobei der retrovirale Vektor ein lentiviraler Vektor ist;

das Poloxamin in einer Konzentration im Bereich von etwa 0,1 bis etwa 40 mg/ml, vorzugsweise von etwa 0,2 bis etwa 30 mg/ml, besonders bevorzugt zwischen etwa 0,3 und etwa 20 mg/ml bereitgestellt wird, wobei

(a) wenn die Zelle eine Zelle in Suspension ist, etwa 5 bis etwa 20 mg/ml bevorzugt wird; und
b) wenn die Zelle anhaftet, etwa 0,3 bis etwa 10 mg/ml bevorzugt wird; und/oder
(a) die polykationischen Polymere ausgewählt sind aus 1,5-Dimethyl-1,5-diaza-undeca-methyl-polymethobromid (Polybren) und Poly(ethylenglykol)-Poly(L-lysin)-Blockcopolymer (PEG-PLL);
b) die polykationischen Peptide ausgewählt sind aus Protaminsulfat, Poly-L-Lysin (PLL), das ein mittleres Molekulargewicht von etwa 1 bis etwa 300 kDa und Vectofusin-1® aufweist;
(c) das Glykoprotein Fibronektin (RetroNectin®) ist;
(d) das Prostaglandin PGE2 ist;
(e) das Poloxamer Synperonic® F108 oder Synperonic® F98 ist; und/oder
(f) das Cyclosporin CsA oder CsH ist,

wobei sich "etwa" auf +/- 30% bezieht.

**15.** Verwendung nach Anspruch 8, wobei

der retrovirale Vektor ein lentiviraler Vektor ist;
das Poloxamin in einer Konzentration im Bereich von etwa 0,1 bis etwa 40 mg/ml, vorzugsweise von etwa 0,2 bis etwa 30 mg/ml, besonders bevorzugt zwischen etwa 0,3 und etwa 20 mg/ml bereitgestellt wird, wobei

(a) wenn die Zelle eine Zelle in Suspension ist, etwa 5 bis etwa 20 mg/ml bevorzugt wird; und
b) wenn die Zelle anhaftet, etwa 0,3 bis etwa 10 mg/ml bevorzugt wird.

(a) die polykationischen Polymere ausgewählt sind aus 1,5-Dimethyl-1,5-diaza-undeca-methyl-polymethobromid (Polybren) und Poly(ethylenglykol)-Poly(L-lysin)-Blockcopolymer (PEG-PLL);
b) die polykationischen Peptide ausgewählt sind aus Protaminsulfat, Poly-L-Lysin (PLL), das ein mittleres Molekulargewicht von etwa 1 bis etwa 300 kDa und Vectofusin-1® aufweist;
(c) das Glykoprotein Fibronektin (RetroNectin®) ist;
(d) das Prostaglandin PGE2 ist;
(e) das Poloxamer Synperonic® F108 oder Synperonic® F98 ist; und/oder
(f) das Cyclosporin CsA oder CsH ist

wobei sich "etwa" auf +/- 30% bezieht.

**16.** Verfahren nach einem der Ansprüche 9 bis 13, wobei

der retrovirale Vektor ein lentiviraler Vektor ist;
das Poloxamin in einer Konzentration im Bereich von etwa 0,1 bis etwa 40 mg/ml, vorzugsweise von etwa 0,2 bis etwa 30 mg/ml, besonders bevorzugt zwischen etwa 0,3 und etwa 20 mg/ml bereitgestellt wird, wobei

(a) wenn die Zelle eine Zelle in Suspension ist, etwa 5 bis etwa 20 mg/ml bevorzugt wird; und
b) wenn die Zelle anhaftet, etwa 0,3 bis etwa 10 mg/ml bevorzugt wird; und/oder

(a) die polykationischen Polymere ausgewählt sind aus 1,5-Dimethyl-1,5-diaza-undeca-methyl-polymethobromid (Polybren) und Poly(ethylenglykol)-Poly(L-lysin)-Blockcopolymer (PEG-PLL);
b) die polykationischen Peptide ausgewählt sind aus Protaminsulfat, Poly-L-Lysin (PLL), das ein mittleres Molekulargewicht von etwa 1 bis etwa 300 kDa und Vectofusin-1® aufweist;
(c) das Glykoprotein Fibronektin (RetroNectin®) ist;
(d) das Prostaglandin PGE2 ist;

(e) das Poloxamer Synperonic® F108 oder Synperonic® F98 ist; und/oder

(f) das Cyclosporin CsA oder CsH ist,

wobei sich "etwa" auf +/- 30% bezieht.

**Revendications**

1. Composition comprenant ou constituée de

   (a) une poloxamine ;
   (b)

      (i) un vecteur rétroviral ; et
      (ii) une cellule de vertébré.

2. Composition selon la revendication 1, dans laquelle

   a) ladite poloxamine est une poloxamine séquentielle ; et/ou
   b) présente une structure de formule (I)

   $$R^1R^2N\text{-}(CH_2)_2\text{-}NR^3R^4 \qquad \text{(I)}$$

   ou de formule (II)

   $$R^1R^2R^5N^+\text{-}(CH_2)_2\text{-}N+R^3R^4R^6 \; C\text{-}I \qquad \text{(II)}$$

   dans laquelle dans l'une ou l'autre formule

   $R^1$ is $H\text{-}(O(CH_2)_2)_a\text{-}(OCH(CH_3)CH_2)_b\text{-}$;
   $R^2$ is $H\text{-}(O(CH_2)_2)_c\text{-}(OCH(CH_3)CH_2)_d\text{-}$;
   $R^3$ is $\text{-}(CH_2CH(CH_3)O)_e\text{-}((CH_2)_2O)_r\text{-}H$;
   $R^4$ is $\text{-}(CH_2CH(CH_3)O)_g\text{-}((CH_2)_2O)_h\text{-}H$;
   $R^5$ et $R^6$ sont indépendamment absents ou sélectionnés parmi H, $C_1$ à $C_6$ alkyl, $C_2$ à $C_6$ alcényle et $C_2$ à $C_6$ alkinyle ; dans laquelle si $R^5$ ou $R^6$ sont absents, le N lié à $R^5$ ou $R^6$ ne porte pas de charge positive ;
   Cl représente un ou plusieurs contre-ions qui rendent la structure de formule (II) électroneutre ;
   a, c, f et h sont indépendamment un entier compris entre environ 25 et environ 200, de préférence entre environ 50 et environ 120 ; et
   b, d, e et g sont indépendamment un entier compris entre environ 5 et environ 50, de préférence entre environ 10 et environ 25, dans laquelle « environ » se réfère à + / - 30%.

3. Composition selon la revendication 2(b), dans laquelle

   a = c ; b = d ; et/ou e = g ; et de préférence
   a = c = f = h ; et/ou b = d = e = g.

4. Composition selon la revendication 2(b) ou 3, dans laquelle le rapport oxyde de propylène - oxyde d'éthylène (# PO/ # EO) défini comme

   $$(b + d + e + g) / (a + c + f + h)$$

   se situe dans la plage d'environ 0,05 à environ 1,5, de préférence d'environ 0,1 à environ 1,0, plus dans les cas plus préférés d'environ 0,15 à environ 0,67, et dans les cas les plus préférés d'environ 0,18 à environ 0,35, dans laquelle « environ » se réfère à +/-30%.

5. Composition selon l'une quelconque des revendications 1 à 4, dans laquelle ladite composition comprend en outre ou est en outre constituée de

(a) une ou plusieurs substances polycationiques choisies parmi le groupe des polymères polycationiques ou des peptides polycationiques ;
(b) des prostaglandines ;
(c) des glycoprotéines ;
(d) des poloxamères ; et/ou
(e) des cyclosporines.

6. Composition pharmaceutique comprenant ou constituée de la composition selon l'une quelconque des revendications 1 à 5.

7. Composition pharmaceutique selon la revendication 6, dans laquelle ledit vecteur rétroviral porte un gène bénéfique pour un individu souffrant d'une maladie ou risquant de développer ladite maladie.

8. Utilisation in vitro ou ex vivo de la poloxamine pour la transduction d'une cellule de vertébré avec un vecteur rétroviral, dans laquelle, optionnellement, on utilise également

(a) une ou plusieurs substances polycationiques choisies parmi le groupe des polymères polycationiques ou des peptides polycationiques ;
(b) des prostaglandines ;
(c) des glycoprotéines ;
(d) des poloxamères ; et/ou
(e) des cyclosporines,

dans laquelle ladite utilisation n'implique pas un procédé de modification de l'identité génétique germinale des êtres humains.

9. Procédé in vitro ou ex vivo de transduction d'une cellule de vertébré avec un vecteur rétroviral, ledit procédé comprenant ou consistant à mettre en contact ladite cellule de vertébré, une poloxamine et ledit vecteur viral,

dans lequel la mise en contact est de préférence effectuée dans l'ordre suivant :

(a) fourniture de ladite cellule ;
b) ajout de ladite poloxamine ; et
(c) ajout dudit vecteur,

dans lequel, de préférence après ladite mise en contact, la spinoculation est effectuée,
dans lequel ledit procédé ne constitue pas un procédé de modification de l'identité génétique germinale des êtres humains.

10. Procédé selon la revendication 9, dans lequel la transduction est améliorée par rapport à un procédé de référence, ce procédé de référence ne comporte pas d'ajout de poloxamine, ledit procédé de référence étant par ailleurs identique audit procédé de transduction d'une cellule de vertébré.

11. Procédé d'augmentation du nombre de copies virales (VCN), ledit procédé comprenant le procédé de la revendication 9, dans lequel le VCN est augmenté par rapport à un procédé de référence, ce procédé de référence ne comporte pas d'ajout de poloxamine, ledit procédé de référence étant par ailleurs identique audit procédé d'augmentation du VCN, dans lequel de préférence l'augmentation du VCN est d'au moins 1,5 fois, d'au moins 2 fois, d'au moins 3 fois, d'au moins 5 fois ou d'au moins 10 fois.

12. Le procédé selon l'une quelconque des revendications 9 à 11, dans lequel

(a) ledit vecteur rétroviral est un vecteur intraviral ; et/ou
(b) ladite poloxamine est T1107.

13. Procédé selon l'une quelconque des revendications 9 à 12, dans lequel ladite mise en contact comprend ou consiste en outre à mettre en contact avec

(a) une ou plusieurs substances polycationiques choisies parmi le groupe des polymères polycationiques ou des peptides polycationiques ;
(b) des prostaglandine ;
(c) des glycoprotéines ;
(d) des poloxamères ; et/ou
(e) des cyclosporines.

14. Composition selon l'une quelconque des revendications 1 à 7, dans laquelle ledit vecteur rétroviral est un vecteur lentiviral ;

ladite poloxamine est fournie à une concentration comprise entre environ 0,1 et environ 40 mg/ml, de préférence entre environ 0,2 et environ 30 mg/ml, dans les cas plus préférés entre environ 0,3 et environ 20 mg/ml, dans lequel

(a) si ladite cellule est une cellule en suspension, environ 5 à environ 20 mg/ml est préféré ; et
(b) si ladite cellule est adhérente, environ 0,3 à environ 10 mg/ml est préféré ; et/ou

(a) lesdits polymères polycationiques sont sélectionnés parmi le 1,5-diméthyl-1,5-diaza-undéca-méthyl-polyméthobromure (Polybrène) et le copolymère séquencé poly(éthylène glycol ) -poly(L-lysine) (PEG-PLL) ;
(b) lesdits peptides polycationiques sont sélectionnés parmi le sulfate de protamine, la poly-L-lysine (PLL) présentant un poids moléculaire moyen d'environ 1 à environ 300 kDa et Vectofusine-1® ;
(c) ladite glycoprotéine est la fibronectine (RetroNectin®) ;
(d) ladite prostaglandine est la PGE2 ;
(e) ledit poloxamère est Synperonic® F108 ou Synperonic® F98 ; et/ou
(f) ladite cyclosporine est CsA ou CsH,

dans lequel « environ » fait à +/- 30 %.

15. Utilisation selon la revendication 8, dans laquelle

ledit vecteur rétroviral est un vecteur lentiviral ;
ladite poloxamine est fournie à une concentration allant d'environ 0,1 à environ 40 mg/ml, de préférence d'environ 0,2 à environ 30 mg/ml, dans les cas plus préférés entre environ 0,3 et environ 20 mg/ml, dans laquelle

(a) si ladite cellule est une cellule en suspension, environ 5 à environ 20 mg/ml est préféré ; et
(b) si ladite cellule est adhérente, environ 0,3 à environ 10 mg/ml est préféré.

(a) lesdits polymères polycationiques sont sélectionnés parmi le 1,5-diméthyl-1,5-diaza-undéca-méthyl-polyméthobromure (Polybrène) et le copolymère séquencé poly(éthylène glycol ) -poly(L-lysine) (PEG-PLL) ;
(b) lesdits peptides polycationiques sont sélectionnés parmi le sulfate de protamine, la poly-L-lysine (PLL) présentant un poids moléculaire moyen d'environ 1 à environ 300 kDa et Vectofusine-1® ;
(c) ladite glycoprotéine est la fibronectine (RetroNectin®) ;
(d) ladite prostaglandine est la PGE2 ;
(e) ledit poloxamère est Synperonic® F108 ou Synperonic® F98 ; et/ou
(f) ladite cyclosporine est CsA ou CsH

dans lequel « environ » fait référence à +/- 30 %.

16. Procédé selon l'une quelconque des revendications 9 à 13, dans lequel

ledit vecteur rétroviral est un vecteur lentiviral ;
ladite poloxamine est fournie à une concentration comprise dans la plage comprise entre environ 0,1 et environ 40 mg/ml, de préférence entre environ 0,2 et environ 30 mg/ml, dans les cas plus préférés entre environ 0,3 et environ 20 mg/ml, dans lequel

(a) si ladite cellule est une cellule en suspension, environ 5 à environ 20 mg/ml est préféré ; et

(b) si ladite cellule est adhérente, environ 0,3 à environ 10 mg/ml est préféré ; et/ou

(a) lesdits polymères polycationiques sont sélectionnés parmi le 1,5-diméthyl-1,5-diaza-undéca-méthyl-polyméthobromure (Polybrène) et le copolymère séquencé poly(éthylène glycol)-poly(L-lysine) (PEG-PLL) ;
(b) lesdits peptides polycationiques sont sélectionnés parmi le sulfate de protamine, la poly-L-lysine (PLL) présentant un poids moléculaire moyen d'environ 1 à environ 300 kDa et Vectofusine-1® ;
(c) ladite glycoprotéine est la fibronectine (RetroNectin®) ;
(d) ladite prostaglandine est la PGE2 ;
(e) ledit poloxamère est Synperonic® F108 ou Synperonic® F98 ; et/ou
(f) ladite cyclosporine est CsA ou CsH,

dans lequel "environ" faite référence à +/- 30 %.

**Figure 1**

**a)**

## Figure 1 continued

b)

| relative to virus only | T908 | T1107 | T1307 |
|---|---|---|---|
| 5,00mg/ml | 1,27 | 1,84 | 1,91 |
| 2,50mg/ml | 1,18 | 2,18 | 2,14 |
| 1,25mg/ml | 1,58 | 2,21 | 2,21 |
| 0,63mg/ml | 2,13 | 2,55 | 2,91 |
| 0,31mg/ml | 1,94 | 1,93 | 2,12 |

# Figure 1 continued

## c)

NCI-H1838

T1107    T1307    T908    controls    - - - - - Virus only

## d)

| relative to virus only | | | |
|---|---|---|---|
| | T1107 | T1307 | T908 |
| 20,00mg/ml | 0,85 | 1,02 | 0,83 |
| 10,00mg/ml | 2,33 | 1,93 | 2,43 |
| 5,00mg/ml | 1,98 | 1,65 | 1,75 |
| 2,50mg/ml | 2,73 | 1,88 | 2,26 |
| 1,25mg/ml | 2,51 | 1,73 | 1,88 |
| 0,63mg/ml | 2,86 | 2,06 | 1,78 |
| 0,31mg/ml | 2,49 | 2,77 | 2,68 |

**Figure 2**

**a)**

**Figure 2 continued**

**b)**

**Figure 2 continued**

**c)**

# Figure 2 continued

## d)

**Figure 2 continued**

**e)**

A2780 - T1107 combinations

**Figure 2 continued**

**f)**

A2780 - T1307 combinations

p-value = 0,001150

**Figure 2 continued**

**g)**

**Figure 3**

**a)**

**Figure 3 continued**

**b)**

# Figure 3 continued

c)

| relative to virus only (without spinoculation) | | | |
|---|---|---|---|
| | T908 | T1107 | T1307 |
| 5,00mg/ml | 3,53 | 4,08 | 3,97 |
| 2,50mg/ml | 3,74 | 5,98 | 4,85 |
| 1,25mg/ml | 2,99 | 4,86 | 4,82 |
| 0,63mg/ml | 2,48 | 4,07 | 3,88 |
| 0,31mg/ml | 1,99 | 3,29 | 4,21 |
| 0,16mg/ml | 1,48 | 1,99 | 2,51 |
| 0,08mg/ml | 1,19 | 1,47 | 1,53 |

d)

| relative to virus only (with spinoculation) | | | |
|---|---|---|---|
| | T908 | T1107 | T1307 |
| 5,00mg/ml | 4,07 | 2,91 | 3,02 |
| 2,50mg/ml | 4,08 | 4,12 | 3,68 |
| 1,25mg/ml | 3,99 | 5,06 | 4,32 |
| 0,63mg/ml | 3,82 | 4,24 | 3,72 |
| 0,31mg/ml | 1,54 | 2,69 | 3,98 |
| 0,16mg/ml | 1,37 | 1,79 | 2,62 |
| 0,08mg/ml | 1,21 | 1,31 | 1,51 |

**Figure 4**

**a)**

**b)**

| relative to virus only | | | |
|---|---|---|---|
| | T908 | T1107 | T1307 |
| **20,00mg/ml** | 2,46 | 2,15 | 2,47 |
| **10,00mg/ml** | 1,75 | 2,17 | 2,07 |
| **5,00mg/ml** | 1,86 | 2,13 | 1,76 |
| **2,50mg/ml** | 1,67 | 1,79 | 1,59 |
| **1,25mg/ml** | 1,63 | 1,44 | 1,44 |
| **0,63mg/ml** | 1,22 | 1,08 | 1,29 |
| **0,31mg/ml** | 1,17 | 0,91 | 1,13 |

## Figure 5

**a)**

**b)**

| relative to virus only | | | |
|---|---|---|---|
| | T908 | T1107 | T1307 |
| 20,00mg/ml | 2,26 | 2,42 | 3,29 |
| 10,00mg/ml | 1,24 | 1,57 | 1,98 |
| 5,00mg/ml | 1,43 | 1,77 | 1,66 |
| 2,50mg/ml | 1,51 | 1,61 | 1,71 |
| 1,25mg/ml | 1,15 | 1,39 | 1,42 |
| 0,63mg/ml | 1,29 | 1,36 | 1,33 |
| 0,31mg/ml | 1,16 | 1,15 | 1,15 |

## Figure 6

### a)

### b)

| | T908 | T1107 | T1307 |
|---|---|---|---|
| **20,00mg/ml** | 75% | 116% | 109% |
| **10,00mg/ml** | 70% | 113% | 99% |
| **5,00 mg/ml** | 69% | 111% | 99% |
| **2,50 mg/ml** | 78% | 110% | 87% |
| **1,25 mg/ml** | 82% | 107% | 93% |
| **0,63 mg/ml** | 86% | 99% | 82% |
| **0,31 mg/ml** | 90% | 99% | 84% |

**Figure 7**

relative transduction enhancement d9/10

**Figure 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017019905 A1
- WO 2013127964 A1
- WO 2013127964 A
- WO 2015104376 A

**Non-patent literature cited in the description**

- **ANA REY-RICO et al.** *Acta Biomaterialia*, 28 August 2015, vol. 27, 42-52
- **MA C. CIUCUREL et al.** Journal of Biomaterials Science. Polymer, 01 January 2011, vol. 22, 2515-2528
- **HOWARD E. DAVIS et al.** *Biophysical Journal*, 01 February 2004, vol. 86 (2), 1234-1242
- **LEE H J et al.** Biologicals. Academic Press LTD, 01 August 2009, vol. 37, 203-209
- **GARRETT C. HEFFNER et al.** *Molecular Therapy: The Journal of the American Society of Gene Therapy*, 01 January 2018, vol. 26 (1), 320-328
- **PETRILLO CAROLINA et al.** *Cell Stem Cell*, 08 November 2018, vol. 23, 820
- **GRETCHEN LEWIS et al.** *Molecular Therapy*, 05 April 2018, vol. 9, 313-322
- **COFFIN JM** ; **HUGHES SH** ; **VARMUS HE**. Retroviruses. Cold Spring Harbour Laboratory Press, 1997
- *Curr Gene Ther.*, 2005, vol. 5, 387-398
- **ALAKHOV, VALERY** ; **EVGUENI KLINSKI** ; **SHENGMIN LI** ; **GRZEGORZ PIETRZYNSKI.** Block Copolymer-Based Formulation of Doxorubicin. From Cell Screen to Clinical Trials.. *Colloids and Surfaces B: Biointerfaces*, 1999, vol. 16, 113-34
- **ALVAREZ-LORENZO, CARMEN et al.** Poloxamine-Based Nanomaterials for Drug Delivery.. *Frontiers in biosciences*, 2010, vol. 2, 424-40
- **ANASTASOV, NATASA et al.** Efficient ShRNA Delivery into B and T Lymphoma Cells Using Lentiviral Vector-Mediated Transfer.. *Journal of hematopathology*, 2009, vol. 2 (1), 9-19
- C/EBPbeta Expression in ALK-Positive Anaplastic Large Cell Lymphomas Is Required for Cell Proliferation and Is Induced by the STAT3 Signaling Pathway.. *Haematologica*, vol. 95 (5), 760-67
- **AUBIN, R J** ; **M WEINFELD** ; **M C PATERSON.** Factors Influencing Efficiency and Reproducibility of Polybrene-Assisted Gene Transfer.. *Somatic cell and molecular genetics*, 1988, vol. 14 (2), 155-67
- **BARTEL, DAVID P.** MicroRNAs: Target Recognition and Regulatory Functions.. *Cell*, 2009, vol. 136 (2), 215-33
- **BISCHOF, DANIELA** ; **KENNETH CORNETTA.** Flexibility in Cell Targeting by Pseudotyping Lentiviral Vectors.. *Methods in molecular biology (Clifton, N.J.)*, 2010, vol. 614, 53-68
- **BUKRINSKY, M I et al.** A Nuclear Localization Signal within HIV-1 Matrix Protein That Governs Infection of Non-Dividing Cells.. *Nature*, 1993, vol. 365 (6447), 666-69
- **BURNS, J C et al.** Vesicular Stomatitis Virus G Glycoprotein Pseudotyped Retroviral Vectors: Concentration to Very High Titer and Efficient Gene Transfer into Mammalian and Nonmammalian Cells.. *Proceedings of the National Academy of Sciences of the United States of America*, 1993, vol. 90 (17), 8033-37
- **CASTRO, B A** ; **C D WEISS** ; **L D WIVIOTT** ; **J A LEVY.** Optimal Conditions for Recovery of the Human Immunodeficiency Virus from Peripheral Blood Mononuclear Cells.. *Journal of clinical microbiology*, 1988, vol. 26 (11), 2371-76
- **CHIAPPETTA, DIEGO A.** ; **ALEJANDRO SOSNIK.** Poly(Ethylene Oxide)-Poly(Propylene Oxide) Block Copolymer Micelles as Drug Delivery Agents: Improved Hydrosolubility, Stability and Bioavailability of Drugs.. *European Journal of Pharmaceutics and Biopharmaceutics*, 2007, vol. 66 (3), 303-17
- **CHO, EUNHEE** ; **JEOUNG SOO LEE** ; **KEN WEBB.** Formulation and Characterization of Poloxamine-Based Hydrogels as Tissue Sealants.. *Acta Biomaterials*, 2012, vol. 8 (6), 2223-32
- **CHU, KANG** ; **KENNETH G CORNETTA** ; **MICHAEL J ECONS.** Efficient and Stable Gene Expression into Human Osteoclasts Using an HIV-1-Based Lentiviral Vector. *DNA and cell biology*, 2008, vol. 27 (6), 315-20
- *Journal of virology*, vol. 72 (11), 8463-71

- **ERUKOVA, VERONIKA YU** ; **OKSANA O. KRYLO-VA** ; **YURI N. ANTONENKO** ; **NICKOLAY S. MELIK-NUBAROV.** Effect of Ethylene Oxide and Propylene Oxide Block Copolymers on the Permeability of Bilayer Lipid Membranes to Small Solutes Including Doxorubicin.. *Biochimica et Biophysica Acta - Bio-membranes*, 2000, vol. 1468 (1-2), 73-86
- **FENARD, DAVID et al.** Vectofusin-1, a New Viral Entry Enhancer, Strongly Promotes Lentiviral Transduction of Human Hematopoietic Stem Cells.. *Molecular Therapy - Nucleic Acids*, 2013, vol. 2, e90
- **FUNKE, SABRINA et al.** Targeted Cell Entry of Lentiviral Vectors.. *Molecular therapy: the journal of the American Society of Gene Therapy*, 2008, vol. 16 (8), 1427-36
- **GRUBER, A et al.** Dendritic Cells Transduced by Multiply Deleted HIV-1 Vectors Exhibit Normal Phenotypes and Functions and Elicit an HIV-Specific Cytotoxic T-Lymphocyte Response in Vitro.. *Blood*, 2000, vol. 96 (4), 1327-33
- **GUNDRY, MICHAEL C** ; **LORENZO BRUNETTI** ; **ANGELIQUE LIN** ; **ALLISON E MAYLE.** Highly Efficient Genome Editing of Murine and Human Hematopoietic Progenitor Cells by CRISPR/Cas9.. *Cell Reports*, 2016, vol. 17 (5), 1453-61
- **GUO, J.** ; **W. WANG** ; **D. YU** ; **Y. WU.** Spinoculation Triggers Dynamic Actin and Cofilin Activity That Facilitates HIV-1 Infection of Transformed and Resting CD4 T Cells. *Journal of Virology*, 2011, vol. 85 (19), 9824-33, http://jvi.asm.org/cgi/-doi/10.1128/JVI.05170-11
- **HANSEN, SCOTT G et al.** Profound Early Control of Highly Pathogenic SIV by an Effector Memory T-Cell Vaccine.. *Nature*, 2011, vol. 473 (7348), 523-27
- **HARADA, ATSUSHI** ; **KAZUNORI KATAOKA.** For-mation of Polyion Complex Micelles in an Aqueous Milieu from a Pair of Oppositely-Charged Block Copolymers with Poly(Ethylene Glycol) Segments.. *Macromolecules*, 1995, vol. 28 (15), 5294-99, https://doi.org/10.1021/ma00119a019
- **HARGUS, GUNNAR et al.** Differentiated Parkinson Patient-Derived Induced Pluripotent Stem Cells Grow in the Adult Rodent Brain and Reduce Motor Asymmetry in Parkinsonian Rats.. *Proceedings of the National Academy of Sciences of the United States of America*, 2010, vol. 107 (36), 15921-26
- **HAUBER, ILONA et al.** Improving Lentiviral Trans-duction of CD34+ Hematopoietic Stem and Progenitor Cells.. *Human Gene Therapy Methods*, 2018, http://www.liebertpub.com/-doi/10.1089/hgtb.2017.085.
- **HEFFNER, GARRETT C. et al.** Prostaglandin E2 Increases Lentiviral Vector Transduction Efficiency of Adult Human Hematopoietic Stem and Progenitor Cells.. *Molecular Therapy*, 2018, vol. 26 (1), 320-28, https://doi.org/10.1016/j.ymthe.2017.09.025.
- **HESSE, J** ; **P EBBESEN** ; **G KRISTENSEN.** Correlation between Polyion Effect on Cell Susceptibility to in Vitro Infection with Murine C-Type Viruses and Polyion Effect on Some Membrane-Related Functions.. *Intervirology*, 1978, vol. 9 (3), 173-83
- **INGRAO, DINA et al.** Concurrent Measures of Fusion and Transduction Efficiency of Primary CD34+ Cells with Human Immunodeficiency Virus 1-Based Lentiviral Vectors Reveal Different Effects of Transduction Enhancers.. *Human Gene Therapy Methods*, 2014, vol. 25 (1), 48-56, http://online.liebertpub.com/doi/10.1089/hgtb.2013.090
- **JACOME, ARIANA et al.** Lentiviral-Mediated Ge-netic Correction of Hematopoietic and Mesenchymal Progenitor Cells from Fanconi Anemia Patients.. *Molecular therapy: the journal of the American Society of Gene Therapy*, 2009, vol. 17 (6), 1083-92
- **JAENISCH, RUDOLF** ; **RICHARD YOUNG.** Stem Cells, the Molecular Circuitry of Pluripotency and Nuclear Reprogramming.. *Cell*, 2008, vol. 132 (4), 567-82
- **KABANOV, ALEXANDER** ; **JIAN ZHU** ; **VALERY ALAKHOV**. Pluronic Block Copolymers for Gene Delivery.. *Advances in genetics*, 2005, vol. 53 (05)
- **KHAN, FAHEEM AHMED et al.** CRISPR / Cas9 Therapeutics: A Cure for Cancer and Other Genetic Diseases.. *Oncotarget*, 2016, vol. 7 (32), 52541-52
- **KOWAL, JOANNA** ; **MERCEDES TKACH** ; **CLO-TILDE THERY.** Biogenesis and Secretion of Exosomes.. *Current Opinion in Cell Biology*, 2014, vol. 29 (1), 116-25
- **LEE, HYUN JOO et al.** Retronectin Enhances Lentivirus-Mediated Gene Delivery into Hematopoie-tic Progenitor Cells.. *Biologicals*, 2009, vol. 37 (4), 203-9, http://dx.doi.org/10.1016/j.biologi-cals.2009.01.008
- **LEMIEUX, P** ; **N GUERIN.** A Combination of Poloxamers Increases Gene Expression of Plasmid DNA in Skeletal Muscle.. *Gene Therapy*, 2000, vol. 7, 986-91
- **LEWIS, GRETCHEN et al.** Staurosporine Increases Lentiviral Vector Transduction Efficiency of Human Hematopoietic Stem and Progenitor Cells.. *Molecular Therapy - Methods and Clinical Development 9(June)*, 2018, 313-22, https://doi.org/10.1016/j.omtm.2018.04.001
- **LIN, HUAN-TING et al.** Application of Droplet Digital PCR for Estimating Vector Copy Number States in Stem Cell Gene Therapy.. *Human gene therapy methods*, 2016, vol. 27 (5), 197-208
- **MAHAJAN, VIVEK et al.** Horizontal Gene Transfer from Macrophages to Ischemic Muscles upon Delivery of Naked DNA with Pluronic Block Copolymers.. *Blomaterials*, 2017, vol. 1 (919), 58-70

- **MARCONI, PEGGY ; ROBERTO MANSERVIGI ; ALBERTO L EPSTEIN.** HSV-1-Derived Helper-Independent Defective Vectors, Replicating Vectors and Amplicon Vectors, for the Treatment of Brain Diseases.. *Current opinion in drug discovery & development*, 2010, vol. 13 (2), 169-83
- **MATRAI, JANKA ; MARINEE K L CHUAH ; THIERRY VANDENDRIESSCHE.** Recent Advances in Lentiviral Vector Development and Applications.. *Molecular therapy: the journal of the American Society of Gene Therapy*, 2010, vol. 18 (3), 477-90
- **MEYER, CONARY et al.** Pseudotyping Exosomes for Enhanced Protein Delivery in Mammalian Cells.. *International Journal of Nanomedicine*, 2017, vol. 12
- **MILLINGTON, MICHELLE et al.** Towards a Clinically Relevant Lentiviral Transduction Protocol for Primary Human CD34 Hematopoietic Stem/Progenitor Cells.. *P/oS one*, 2009, vol. 4 (7), e6461
- **MOHR, CHRISTIAN ANDREAS et al.** A Spread-Deficient Cytomegalovirus for Assessment of First-Target Cells in Vaccination.. *Journal of virology*, 2010, vol. 84 (15), 7730-42
- **MORRELL, NATHAN T. et al.** Liposomal Packaging Generates Wnt Protein with in Vivo Biological Activity.. *PLoS ONE*, 2008, vol. 3 (8)
- **MOULAY, GILLES et al.** Histidine-Rich Designer Peptides of the LAH4 Family Promote Cell Delivery of a Multitude of Cargo.. *Journal of Peptide Science*, 2017, vol. 23 (4), 320-28
- **MURRAY, LESLEY et al.** Optimization of Retroviral Gene Transduction of Mobilized Primitive Hematopoietic Progenitors by Using Thrombopoietin, Flt3, and Kit Ligands and RetroNectin Culture.. *Human Gene Therapy*, 1999, vol. 10, 1743-52
- **O'CONNELL, RYAN M et al.** Lentiviral Vector Delivery of Human Interleukin-7 (HIL-7) to Human Immune System (HIS) Mice Expands T Lymphocyte Populations.. *PloS one*, 2010, vol. 5 (8), e12009
- **O'LOUGHLIN, AISLING ; CAROLINE WOFFINDALE ; MATTHEW WOOD.** Exosomes and the Emerging Field of Exosome-Based Gene Therapy.. *Current Gene Therapy*, 2012, vol. 12 (4), 262-74, http://www.eurekaselect.com/openurl/content.php?genre=article&issn=1566-5232&volume=12&issue=4&spage=262.
- **POCZOBUTT, JOANNA M et al.** Benign Mammary Epithelial Cells Enhance the Transformed Phenotype of Human Breast Cancer Cells.. *BMC cancer*, 2010, vol. 10, 373
- **RICCIOTTI, EMANUELE ; FITZGERALD, GARRET A.** Prostaglandins and Inflammation.. *Arterioscler Thromb Vasc Boil*, 2011, vol. 31 (5), 986-1000, https://doi.org/10.1161/ATVBAHA.110.207449
- **ROBERT, MARC-ANDRE et al.** Virus-Like Particles Derived from HIV-1 for Delivery of Nuclear Proteins: Improvement of Production and Activity by Protein Engineering.. *Molecular biotechnology*, 2017, vol. 59 (1), 9-23
- **ROUAS, REDOUANE et al.** Lentiviral-Mediated Gene Delivery in Human Monocyte-Derived Dendritic Cells: Optimized Design and Procedures for Highly Efficient Transduction Compatible with Clinical Constraints. *Cancer gene therapy*, 2002, vol. 9 (9), 715-24
- **SCHMOLKA, IRVING R.** A Review of Block Polymer Surfactants.. *Journal of the American Oil Chemists' Society*, 1977, vol. 54 (3), 110-16
- **SCHWARZBAUER, JEAN E. ; DOUGLAS W. DESIMONE.** Fibronectins, Their Fibrillogenesis, and in Vivo Functions.. *Cold Spring Harbor Perspectives in Biology*, 2011, vol. 3 (7), 1-19
- **SERRA-GÓMEZ, RAFAEL ; CECILE A. DREISS ; JAVIER GONZALEZ-BENITO ; GUSTAVO GONZALEZ-GAITANO.** Structure and Rheology of Poloxamine T1107 and Its Nanocomposite Hydrogels with Cyclodextrin-Modified Barium Titanate Nanoparticles.. *Langmuir*, 2016, vol. 32 (25), 6398-6408
- **SONG, HONGMEI et al.** Pluronic L64-Mediated Stable HIF-1 a Expression in Muscle for Therapeutic Angiogenesis in Mouse Hindlimb Ischemia.. *International journal of nanomedicine*, 2014, vol. 9, 3439-52
- **SUBBARAMAN, LAKSHMAN N et al.** Rewetting Drops Containing Surface Active Agents Improve the Clinical Performance of Silicone Hydrogel Contact Lenses.. *Optometry and vision science: official publication of the American Academy of Optometry*, 2006, vol. 83 (3), 143-51
- **TAKAHASHI, KAZUTOSHI ; SHINYA YAMANAKA.** Induction of Pluripotent Stem Cells from Mouse Embryonic and Adult Fibroblast Cultures by Defined Factors.. *Cell*, 2006, vol. 126 (4), 663-76
- Potent and Specific Inhibitors of Protein Kinase C of Microbial Origin.. **TAMAOKI, T ; H NAKANO.** Bio/technology. Nature Publishing Company, 1990, vol. 8, 732-35
- **TONGE, S ; L JONES ; S GOODALL ; B TIGHE.** The Ex Vivo Wettability of Soft Contact Lenses.. *Current eye research*, 2001, vol. 23 (1), 51-59
- **TILYSUZ, NESRIN et al.** Lipid-Mediated Wnt Protein Stabilization Enables Serum-Free Culture of Human Organ Stem Cells.. *Nature Communications*, 2017, vol. 8, 14578, http://www.ncbi.nlm.nih.gov/pubmed/28262686%0Ahttp://www.nature.com/doifinder/10 .1038/ncomms14578.
- **WINGERATH, JESSICA et al.** Recombinant LCMV Vectors Induce Protective Immunity Following Homologous and Heterologous Vaccinations.. *Molecular therapy: the journal of the American Society of Gene Therapy*, 2017, vol. 25 (11), 2533-45

- **WURM, MELANIE et al.** The Influence of Semen-Derived Enhancer of Virus Infection on the Efficiency of Retroviral Gene Transfer.. *The journal of gene medicine*, 2010, vol. 12 (2), 137-46

- **ZHANG, BINGQI ; MATHUMAI KANAPATHIPILLAI ; PAUL BISSO ; SURYA MALLAPRAGADA.** Novel Pentablock Copolymers for Selective Gene Delivery to Cancer Cells.. *Pharmaceutical Research*, 2009, vol. 26 (3), 700-713